# EUROPEAN PATENT SPECIFICATION

(11) **EP 1 843 755 B1**
(45) Date of publication and mention of the grant of the patent: **01.04.2015**
(21) Application number: 06720247.3
(22) Date of filing: 01.02.2006
(51) Int. Cl.: A61K 9/00, A61K 9/20, A61K 9/16

(54) **GASTRIC RETENTION AND CONTROLLED RELEASE DELIVERY SYSTEM**
SYSTEM ZUR MAGENRETENTION UND ZUR GESTEUERTEN FREISETZUNG
SYSTEME D'ADMINISTRATION A RETENTION GASTRIQUE ET A LIBERATION LENTE

(30) Priority: 01.02.2005 US 649436 P
(43) Date of publication of application: 17.10.2007
(73) Proprietor: Emisphere Technologies, Inc., Cedar Knolls, New Jersey 07927 (US)
(72) Inventor: LIAO, Jun, Yorktown Heights, NY 10598 (US); LIU, Puchun, Chappaqua, NY 10514 (US); DINH, Steven, Briarcliff Manor, NY 10510 (US); SINGH, Brahma, Jamaica, NY 11432 (US); MAJURU, Shingai, Brewster, NY 10509 (US); BHARGAVA, Prateek, N., White Plains, NY 10603 (US); DHOOT, Nikhil, Dombivli (East) 421 203 (IN)
(74) Representative: Grünecker, Kinkeldey, Stockmair & Schwanhäusser
(86) International application number: PCT/US2006/003899
(87) International publication number: WO 2006/084164

(56) References cited:
- EP-A- 1 424 069
- WO-A-00/48589
- WO-A-98/05362
- WO-A-98/11879
- WO-A-03/035041
- WO-A-03/051304
- WO-A-2004/080401
- US-A1- 2003 152 622
- US-A1- 2004 001 887
- US-A1- 2004 180 088
- US-B1- 6 797 283
- LEE, J. W. ET AL: "Bioadhesive -based dosage forms: the next generation" J. PHARM. SCIE., vol. 89, no. 7, 2000, XP002465247
- KIM, B-Y ET AL: "Bioadhesive interaction and hypoglycemic effect of insulin-loaded lectin-microparticle conjugates in oral insulin delivery system" J. CONTR. RE., vol. 102, 2004, XP002465248
- ANDRIANOV, A.K. ET AL: "Polymeric carriers for oral uptake of microparticulates" ADV. DR. DEL. REV., vol. 34, 1998, XP002465249
- GONZE, M.D. ET AL: "Orally administered heparin for preventing deep venous thrombosis" AM. J. SURG., vol. 176, 1998, XP002465250
- PINEO, G. ET AL: "Oral delivery of heparin: SNAC and related formulations" B. PRAC. RES. CLIN. HEM., vol. 17, no. 1, 2004, pages 153-160, XP002465251

## Description

### FIELD OF THE INVENTION

The present invention relates to pharmaceutical compositions containing a gastric retention and/or controlled release delivery system that includes a delivery agent compound.

### BACKGROUND OF THE INVENTION

Conventional means for delivering drugs are often severely limited by biological, chemical, and physical barriers. Typically, these barriers are imposed by the environment through which delivery occurs, the environment of the target for delivery, and/or the target itself. Examples of physical barriers include the skin, lipid bi-layers and various organ membranes that are relatively impermeable to certain drugs but must be traversed before reaching a target, such as the circulatory system. Chemical barriers include, but are not limited to, pH variations in the gastrointestinal (GI) tract and degrading enzymes. These barriers are of particular significance in the design of oral delivery systems. Oral delivery of many drugs often requires greater amounts of drug to be administered than if the drug were administered by a different route.

In addition to these physical barriers, there are barriers with regard to site of active agent absorption. Certain agents are only absorbed in the stomach or in the small intestine and the passage of particles through this area is generally complete within three to five hours, regardless of particle size, dosage form (e.g. liquid, microencapsulated) or presence of food. This transit time may provide a window of opportunity that is too short to facilitate the absorption of therapeutic quantities of active agent. Such agents require administration of frequent doses, an inconvenience and expense to patients and clinicians, and which often results in non-compliance by the patient and failure of therapy.

Controlled release dosage forms typically provide prolonged release of active agents and constant rate of delivery of active agents. However, it is often preferable to have deliver the active agent to a targeted site or sites, such as the stomach, duodenum or small intestine.

Previous patents and published applications describe several formulations which are retained in the stomach for prolonged periods of time. See, for example, U.S. Pat. No. 6,797,283, U.S. Pat. No. 4,851,232, U.S. Pat. No. 4,871,548, U.S. Pat. No. 4,767,627, U.S. Pat. No. 5,443,843, U.S. Pat. No. 5,007,790, U.S. Pat. No. 5,582,837, International Published Application No. WO 99/07342, U.S. Patent No. 4,290,426 U.S. Patent No. 5,256,440, U.S. Patent No. 4,839,177, U.S. Patent No. 5,780,057, U.S. 5,534,263, and U.S. Patent Nos. 3,845,770, 3,995,631, 4,034,756, 4,111,202, 4,320,755, 4,327,725, 4,449,983, 4,765,989, 4,892,778, 4,940,465, 4,915,949, and 5,126,142.

WO 00/48589 relates to solid oral dosage forms comprising heparin in combination with a carrier; WO 2004/080401 is directed to oral insulin therapies; EP 1424069 describes drug dosage forms that are retained in the stomach and gradually deliver sparingly soluble drugs or insoluble, particulate matter over a time period of several hours; US 2004/0001887 relates to bioadhesive, bioerodible compositions for the extended and controlled release of active agents; US 2003/0152622 relates to controlled release oral dosage forms of diuretics; WO 03/035041 describes gastric retentive oral dosage forms with restricted drug release in the lower gastrointestinal tract. Lee, J.W. et al. "Bioadhesive-based dosage forms: The next generation." Journal of Pharmaceutical Sciences. 89, 7(2000): 850-866, describes bioadhesive based dosage forms. US 2004/0180088 is directed to a gastric retention controlled drug delivery system having a biphasic release pattern. Makeni Chemicals Ltd. Technical Data Sheet of Kollidon® 90F (2004), discloses polyvinylpyrrolidone as a binder in granules and tablets. Bühler, V. "Kollidon®: Polyvinylpyrrolidone for the pharmaceutical industry." BASF 4th Ed. (1998): 36, discloses the molecular weight of Kollidon®. Chawla, G., et al. "Gastroretention: A means to address regional variability in intestinal drug absorption." Pharmaceutical Technology July, 2003: 50-68, describes gastroretentive drug delivery systems to improve the bioavailability of drugs that exhibit site-specific absorption.

There remains a need for oral pharmaceutical formulations of active agents which provide prolonged and controlled delivery to areas of the gastrointestinal tract, particularly for agents which need to be retained in the stomach and/or which are not normally bioavailable by the oral route.

### SUMMARY OF THE INVENTION

The problem of the present invention is solved on the basis of claims 1 to 14. The present invention provides a bi-layered pharmaceutical composition comprising one layer comprising a biologically active agent, a delivery agent compound,
a release controlling polymer, and a second layer comprising a swellable polymer. The active agent which is incorporated into pharmaceutical compositions of the present invention is heparin, and the delivery agent compound is N-(8-[2-hydroxybenzoyl]-amino) caprylic acid, or a pharmaceutically acceptable salt thereof. The pharmaceutical compositions containing a swellable polymer are retained in the stomach for an extended period of time, thereby delivering more active agents through the stomach than a similar composition without the swellable polymer or microadhesive. Because active agents in the presence of a delivery agent compound are generally better absorbed in the stomach than other areas of the gastrointestinal tract, retention of the pharmaceutical composition in the stomach results in improved absorption and bioavailability of the active agent.

Preferably, the pharmaceutical formulation is orally administered. For example, the oral pharmaceutical formulations of the present invention may be administered once-a-day, once-a-week, or once-a-month. In other embodiments, the formulations can be administered more frequently, for example twice a day, three times a day, or four times a day.

An embodiment of the present invention provides an oral pharmaceutical formulation which is a bi-layered formulation, such as a tablet or caplet, comprising a therapeutically effective amount of an active agent, and at least one delivery agent. One layer contains the active agent, the delivery agent and a release-controlling polymer (e.g., a polyethylene oxide, preferably having a molecular weight of about 200,000). The second layer contains a swellable polymer (e.g. polyethylene oxide preferably having a molecular weight of about 7,000,000). In various embodiments, this formulation provides, upon ingestion by a human, one or more of the following:
(a) active agent absorption beginning in approximately 15 to 30 minutes from administration lasting at least about 1.5 hours, about 3.0 hours, or about 6.0 hours after administration;
(b) a dosage form that approximately increases in size by at least about 10 or 15%, approximately doubles in size, while in the stomach within 30 minutes of administration;
(c) provides a sustained active agent release profile for the majority of the duration while the dosage remains in the stomach; or
(d) remains in the stomach for at least 4 hours, 6 hours, or 12 hours and/or up to 24 hours, preferably while remaining substantially intact.

One embodiment of the present invention provides a pharmaceutical formulation comprising (a) a first layer containing a pharmaceutically acceptable active agent, metabolite or prodrug thereof, at least one delivery agent and a release controlling polymer and (b) a second layer comprising a swellable polymer, said swellable polymer being in an amount sufficient to swell to an acceptable size for retention within the stomach for up to 1.5 hours, or up to 3 hours, or up to 6 hours. Further embodiments of the present invention may contain a hydroattractant.

In one embodiment, the swellable polymer is poly(ethylene oxide) preferably having a molecular weight of about 4,000,000 to about 9,000,000 dattons, (e.g., 7,000,000 dattons). In one embodiment, the release controlling polymer is poly(ethylene oxide) preferably having a molecular weight of about 100,000 - 300,000 dattons (e.g., 200,000 dattons).

### DETAILED DESCRIEPTION OF THE INVENTION

### Definitions

The term "about" or "approximately" means within an acceptable error range for the particular value as determined by one of ordinary skill in the art, which will depend in part on how the value is measured or determined, i.e., the limitations of the measurement system. For example, "about" can mean within 1, or more than 1 standard deviations, per practice in the art. Alternatively, "about" with respect to the formulations can mean a range of up to 10%, preferably up to 5%.

The terms "alkyl", "alkenyl", "alkoxy", "alkylene", "alkenylene", "alkyl(arylene)", and "aryl(alkylene)" include linear and branched alkyl, alkenyl, alkoxy, alkylene, alkenylene, alkyl(arylene), and aryl(alkylene) groups, respectively.

The phrase "pharmaceutically acceptable" refers to compounds or compositions that are physiologically tolerable and do not typically produce an allergic or similar untoward reaction, such as gastric upset, dizziness, when administered to a mammal.

The term "active agent" as used herein includes racemic as well as its optically pure enantiomers. The term "active agent" also includes solvates, active metabolites, prodrugs, and all pharmaceutically acceptable complexes and hydrates thereof.

An "effective amount of active agent" means the amount of active agent, salt or salts, their solvates, active metabolites, prodrugs, or racemates or enantiomers thereof that, when administered to a mammal for treating or preventing a state, disorder or condition is sufficient to effect such treatment or prevention. The "effective amount" will vary depending on the active ingredient, the state, disorder, or condition to be treated and its severity, and the age, weight, physical condition and responsiveness of the mammal to be treated. An "effective amount of delivery agent" refers to an amount of the delivery agent that promotes the absorption of a desired amount of the active agent from, for example, the gastrointestinal tract.

An "effective amount of the pharmaceutical formulation" is an amount of the pharmaceutical formulation described which is effective to treat or prevent a condition in a subject to whom it is administered over some period of time, e.g., provides a therapeutic effect during a desired dosing interval. Generally, an effective amount of the pharmaceutical formulation includes amounts of active agent, which when administered with at least one delivery agent, treats or prevents the desired condition over a desired period of time (i.e., an effective amount of delivery agent and an effective amount of active agent).

As used herein, the term "treat" includes one or more of the following:
(a) arresting, delaying the onset (i.e., the period prior to clinical manifestation of a disorder) and/or reducing the risk of developing or worsening a disorder;
(b) relieving or alleviating at least one symptom of a disorder in a mammal; or
(c) relieving or alleviating the intensity and/or duration of a manifestation of a disorder experienced by a mammal including, those which are in response to a given stimulus (e.g., pressure, tissue injury or cold temperature). The term "treat" also includes prophylactically preventing, curing, healing, alleviating, relieving, altering, remedying, ameliorating, improving, or affecting a condition (e.g., a disease), the symptoms of the condition, or the predisposition toward the condition.

The terms "sustained release" "extended release" or "long acting" as used herein refers to the release of an active ingredient over an extended period of time leading to lower peak plasma concentrations and a prolonged Tₘₐₓ as compared to "immediate release" or "regular release" formulations of the same active ingredient.

The term "bioavailability" refers to the rate and extent to which the active ingredient or active moiety is absorbed from a drug product and becomes systematically available.

The term "polymorph" refers to crystallographically distinct forms of a substance.

The term "hydrate" as used herein includes, (i) a substance containing water combined in the molecular form and (ii) a crystalline substance containing one or more molecules of water of crystallization or a crystalline material containing free water.

The term "SNAC" as used herein refers to N-(8-[2-hydroxybenzoyl]-amino) caprylic acid and pharmaceutically acceptable salts thereof, including its monosodium and disodium salt. The term "SNAC free acid" refers to N-(8-[2-hydroxybenzoyl]-amino) caprylic acid. Unless otherwise noted, the term "SNAC" refers to all forms of SNAC, including all amorphous and polymorphic forms of SNAC, such as SNAC trihydrate and those described in U.S. Provisional Application Nos. 60/619,418 and 60/569,476. The term "SNAC trihydrate" as used herein refers to a crystalline form of SNAC in which three molecules of water are associated with each molecule of SNAC. SNAC can be prepared, for example, by the procedures described in U.S. Patent No. 5,650,386 and International Publication Nos. WO00/46182 and WO00/59863.

The term "SNAD" as used herein refers to N-(8-[2-hydroxybenzoyl]-amino) decanoic acid and pharmaceutically acceptable salts thereof, including its monosodium salt. Unless otherwise noted, the term "SNAD" refers to all forms of SNAD, including all amorphous and polymorphic forms of SNAD.

The term "4-MOAC" refers to 8-(N-2-hydroxy-4-methoxybenzoyl)-aminocaprylic acid and pharmaceutically acceptable salts thereof. Unless otherwise noted, the term "4-MOAC" refers to all forms of 4-MOAC, including all amorphous and polymorphic forms of 4-MOAC.

The term "5-CNAC" refers to N-(8-[2-hydroxy-5-chlorobenzoyl]-amino)octanoic acid (also known as 8-(N-2-hydroxy-5-chlorobenzoyl)aminocaprylic acid)) and pharmaceutically acceptable salts thereof, including its monosodium salt. Unless otherwise noted, the term "5-CNAC" refers to all forms of 5-CNAC, including all amorphous and polymorphic forms of 5-CNAC.

The term "4-CNAB" refers to 4-[(2-hydroxy-4-chlorobenzoyl)amino]butanoate (also known as 4-[(4-chloro-2-hydroxy-benzoyl)amino]butanoic acid) and pharmaceutically acceptable salts thereof, including its monosodium salt. Unless otherwise noted, the term "4-CNAB" refers to all forms of 4-CNAB, including all amorphous and polymorphic forms of 4-CNAB. The term "sodium 4-CNAB" and "mono-sodium 4-CNAB" refer to monosodium 4-[(2-hydroxy-4-chlorobenzoyl)amino]butanoate, including anhydrous, monohydrate, and isopropanol solvates thereof and amorphous and polymorphic forms thereof (including those described in International Publication No. WO 03/057650), unless otherwise indicated.

The term "solvate" as used herein includes, a molecular or ionic complex of molecules or ions of a solvent with molecules or ions of a delivery agent or active agent.

The term "delivery agent" refers to any of the delivery agent compounds disclosed or incorporated by reference herein.

The term "release controlling polymer" includes polymers, preferably of low to medium molecular weight which allow gradual surface erosion of the active agent-delivery agent complex, thus permitting this complex to enter the stomach and/or the small intestines intact, and hence permitting the active agent to enter the systemic circulation. These polymers should be slightly water soluble and allow water to enter the active agent-delivery agent complex. Representative polymers include, (poly(ethylene oxide) and low molecular weight cellulose derivatives, such as Klucel.

The term "swellable polymer" refers to polymers of high molecular weight and having preferably strong resistance to the shear forces of the digestive processes of the stomach, which expand when orally consumed to provide gastric retention.

### Gastro-Retentive Drug Delivery System

A Gastro-retentive drug delivery system (GRDDS) may also be referred to as a gastric retention dosage form or device. It often incorporates a controlled delivery system that can retain in the stomach for a prolonged time period, typically from 4 hours to 24 hours, during which it continuously releases the active agent(s) to the stomach in a controlled manner. The released active agents may be absorbed in the stomach or dispersed from the stomach to the duodenum or small intestine where they can be absorbed.

GRDDS can increase absorption and improve the therapeutic effect of drugs characterized by a limited and narrow absorption window at the upper part of the GI tract, as well as in drugs intended to treat local diseases in the stomach and the duodenum. Such diseases include gastric ulcers, chemo-induced and radiation-induced mucocytis or infection with a microorganism, such as *Heliocobacter pylori.* These delivery forms might be used to target and retain chemotherapeutic agents in the stomach, upper gastrointestinal tract, and associated organs (e.g. pancreas, liver), thereby increasing the efficacy of cancer treatment in these areas. In addition, the controlled release and retention delivery form could be useful for diagnostic purposes, and used to deliver barium sulfate, other radio-opaque dyes or radioactive tracers, such as I₁₃₁, gallium salts.

Conventional oral dosage forms traverse along GI tract and provide a specific drug concentration in systemic circulation without offering any control over drug delivery. The site of drug delivery is uncertain. Compared to the conventional dosage form, GRDDS is generally a more controlled-release drug delivery system. The site of drug delivery is localized in the stomach and drug release is often designed to occur in a controlled manner. These advantages become even more significant for a drug that has relatively a narrow absorption window. Compared to negligible absorption of the drug released from a conventional dosage form in the region preceding the absorption window, dissolved drug is continuously released from the GRDDS in the stomach and continuously absorbed through the absorption window, resulting in a much longer absorption time and thus higher drug bioavailability. Gastrointestinal motility pattern affects the gastric retention of GRDDS. Two distinct patterns exist, corresponding to the fasted and fed states. The fed state is induced immediately after food ingestion and persists as long as food remains, typically three to four hours. Food is mixed and partially digested. As the stomach undergoes contractions, the digested material is discharged into small intestinal and the non-digested food is retropelled for further digestion. At the end of the digestive period, the stomach enters the fasting state. In the fasting state, the stomach begins a cycle called IMMC (Inter-digestive Migrating Motor Complex), which includes four phases. The total cycle time is about one to two hours. All the contents if not too large are swept out of the stomach by the intense contractions (housekeeper waves) that occur during Phase III.

A gastric retention dosage form can be designed based on a variety of mechanisms such as buoyancy, size, and bio-adhesion, to achieve gastric retention. Floating systems have sufficient buoyancy to float over gastric contents and remain in the stomach for a prolonged period. Bio/mucoadhesive systems adhere to gastric epithelial cell surface, or mucin, and extend the gastric retention by increasing the intimacy and duration of contact between the GRDDS and the biological membrane.

High density systems, which have a density of ~ 3 g/cm³, are retained in the body of the stomach, which is anatomically lower than pyloric sphincter, and are capable of withstanding its peristaltic movements. The threshold density for such GRDDS is 2.4 - 2.8 g/cm³. Swelling/Expandable systems are formulated with expandable polymers. For easy administration, they are fabricated into reasonably small dosage forms. Upon contact with gastric fluid, the polymer imbibes water and swells to a large size that prevents the GRDDS from passing through the pylorus. Sustained / controlled release may be achieved by selecting a polymer with proper molecular weight and swelling properties. The swollen system will eventually lose its integrity because of loss in mechanical strength caused by abrasion or erosion. They may dissolve, erode or disintegrate into small fragments in the presence of gastric juice. Upon completion of releasing the drug, they will be completely and safely eliminated from the GI tract by the body.

### Delivery Agent Compounds

In the present invention, the delivery agent is SNAC. In one embodiment, the delivery agent is a sodium salt of SNAC. In one embodiment, the delivery agent is the disodium salt of SNAC.

Delivery agents of the present invention are also described in U.S. Published Application Nos. 20040110839, 20040106825, 20040068013, 20040062773, 20040022856, 20030235612, 20030232085, 20030225300, 20030198658, 20030133953, 20030078302, 20030072740, 20030045579, 20030012817, 20030008900, 20020155993, 20020127202, 20020120009, 20020119910, 20020102286, 20020065255, 20020052422, 20020040061, 20020028250, 20020013497, 20020001591, 20010039258, and 20010003001. Delivery agents of the present invention are also described in International Publication Nos. WO 2004/4104018, WO 2004080401, WO 2004062587, WO 2003/057650, WO 2003/057170, WO 2003/045331, WO 2003/045306, WO 2003/026582, WO 2002/100338, WO 2002/070438, WO 2002/069937, WO 02/20466, WO 02/19969, WO 02/16309, WO 02/15959, WO 02/02509, WO 01/92206, WO 01/70219, WO 01/51454, WO 01/44199, WO 01/34114, WO 01/32596, WO 01/32130, WO 00/07979, WO 00/06534, WO 00/06184, WO 00/59863, WO 00/59480, WO 00/50386, WO 00/48589, WO 00/47188, WO 00/46182, WO 00/40203, WO 99/16427, WO 98/50341, WO 98/49135, WO 98/34632, WO 98/25589, WO 98/21951, WO 97/47288, WO 97/31938, WO 97/10197, WO 96/40076, WO 96/40070, WO 96/39835, WO 96/33699, WO 96/30036, WO 96/21464, WO 96/12475, and WO 9612474.

The delivery agent compounds depicted as carboxylic acids may be in the form of the carboxylic acid or salts thereof. Suitable salts include organic and inorganic salts, for example alkali-metal salts, such as sodium (e.g., monosodium and disodium salts), potassium and lithium; alkaline-earth metal salts, such as magnesium, calcium or barium; ammonium salts; basic amino acids, such as lysine or arginine; and organic amines, such as dimethylamine or pyridine. Preferably, the salts are sodium salts. The salts may be mono- or multi-valent salts, such as monosodium salts and di-sodium salts. The salts may also be solvates, including ethanol solvates, and hydrates.

The delivery agent compounds depicted as amines may be in the form of the free amine or salts thereof. Suitable salts include organic and inorganic salts, for example sodium salts, sulfate salts, hydrochloride salts, phosphate salts, fluoride salts, carbonate salts, tartrate salts, oxalates, oxides, formates, acetate or citrate.

Salts of the delivery agent compounds of the present invention may be prepared by methods known in the art. For example, sodium salts may be prepared by dissolving the delivery agent compound in ethanol and adding aqueous sodium hydroxide.

Where the delivery agent has an amine moiety and a carboxylic acid moiety, poly amino acids and peptides comprising one or more of these compounds may be used. An amino acid is any carboxylic acid having at least one free amine group and includes naturally occurring and synthetic amino acids. Poly amino acids are either peptides (which are two or more amino acids joined by a peptide bond) or are two or more amino acids linked by a bond formed by other groups which can be linked by, e.g., an ester or an anhydride linkage. Peptides can vary in length from dipeptides with two amino acids to polypeptides with several hundred amino acids. One or more of the amino acids or peptide units may be acylated or sulfonated.

The compounds described herein may be derived from amino acids and can be readily prepared from amino acids by methods within the skill of those in the art, such as those described in International Publication Nos. WO96/30036, WO97/36480, WO00/06534, WO00/46812, WO00/50386, WO00/59863, WO 01/32596, and WO 00/07979 and U.S. Patent Nos. 5,643,957, 5,650,386, and 5,866,536.

For example, the compounds may be prepared by reacting the single amino acid with the appropriate acylating or amine-modifying agent, which reacts with a free amino moiety present in the amino acid to form amides. Protecting groups may be used to avoid unwanted side reactions as would be known to those skilled in the art. With regard to protecting groups, reference is made to T.W. Greene, Protecting Groups in Organic Synthesis, Wiley, New York (1981).

The delivery agent compound may be purified by recrystallization or by fractionation on one or more solid chromatographic supports, alone or linked in tandem. Suitable recrystallization solvent systems include acetonitrile, methanol, ethanol, ethyl acetate, heptane, water, tetrahydrofuran, and combinations thereof. Fractionation may be performed on a suitable chromatographic support such as alumina, using methanol/n-propanol mixtures as the mobile phase; reverse phase chromatography using trifluoroacetic acid/acetonitrile mixtures as the mobile phase; and ion exchange chromatography using water or an appropriate buffer as the mobile phase. When anion exchange chromatography is performed, preferably a 0-500 mM sodium chloride gradient is employed.

### Active Agents

The biologically active agent of the present invention is heparin.

### Swellable Polymers

In embodiments of the present invention, a pharmaceutical composition comprises an active agent, a delivery agent and at least one swellable polymer.

A swellable polymer is a polymer that expands upon ingestion such that the pharmaceutical composition is retained in the stomach for 30 minutes, 90 minutes, 4 hours, 6 hours, 12 hours or 24 hours or more after administration. For example, the swellable polymer may cause the pharmaceutical composition to increase in size 10%, 15%, 50%, 100% or 200% or more as compared to its pre-ingested volume.

Generally higher molecular weights of the polymers are more desirable since they provide a faster swelling rate, larger swollen size and stronger mechanic strength. In one embodiment of the present invention, the swellable polymers has a molecular weight in excess of 50,000 daltons. In another embodiment, the swellable polymer has a molecular weight in excess of 200,000 daltons. In another embodiment, the swellable polymer has a molecular weight in excess of 7,000,000 daltons.

Swellable polymers include a crosslinked poly(acrylic acid), a poly(alkylene oxide), a poly(vinyl alcohol), a poly(vinyl pyrrolidone); a polyurethane hydrogel, a maleic anhydride polymer, such as a maleic anhydride copolymer, a cellulose polymer, a polysaccharide, starch, and starch based polymers.

Examples of poly(alkylene oxides) include polymers which contain as a unit, ethylene oxide, propylene oxide, ethylene oxide, or propylene oxide. These polymers may consist entirely of any of the above units (as a monomer), combinations of any of the above units, such as a copolymer. In one embodiment, the swellable polymer is a block copolymer in which one of the repeating units consists of ethylene oxide, propylene oxide, ethylene oxide, or propylene oxide.

Examples of cellulose polymers include cellulose, hydroxymethylcellulose, hydroxyethyl cellulose, hydroxypropyl cellulose, hydroxypropylmethyl cellulose (also known as hypromellose), and carboxymethyl cellulose.

Examples of polysaccharides include dextran, xanthan gum, gellan gum, welan gum, rhamsan gum, sodium alginate, calcium alginate, chitosan, gelatin, and maltodextrin.

Examples of starch based polymers include hydrolyzed starch polyacrylonitrile graft copolymers, starch-acrylate-acrylamide copolymers.

Commercially available swellable polymers include PolyOX 303™ (Poly(ethylene oxide), molecular weight 7,000,000); PolyOX WSR N-12K (Poly(ethylene oxide), molecular weight 1,000,000), PolyOX WSR N-60K, (Poly(ethylene oxide), molecular weight 2,000,000), PolyOX WSR 301 (Poly(ethylene oxide), molecular weight 4,000,000), PolyOX WSR Coagulant, PolyOX WSR 303, PolyOX WSR 308, NFgrade™ (Poly(ethylene oxide) molecular weight 1,000,000); PolyOX WSR N80™ (Poly(ethylene oxide), molecular weight 200,000); Methocel F4M™ (hydroxypropyl methylcellulose); Methocel A15C (methylcellulose), Methocel A18M™, Methocel K4M™ (hydroxypropyl methylcellulose 2208), Methocel K100™ (hydroxypropyl methylcellulose 2910) Methocel E10M™ (hydroxypropyl methylcellulose 2910), Methocel E4M™ (hydroxypropyl methylcellulose); Methocel K15MP™ (hydroxypropyl methylcellulose); each available from Dow Chemical Company, Midland MI.

Other examples of commercially available swellable polymers include BLANOSE® cellulose gum, including Blanose cellulose gum, grad 7H4 (sodium carboxymethyl cellulose), ECN7 Pharmaceutical Grade™ (Ethyl cellulose); and ECN22 Pharmaceutical Grade™ (Ethylcellulose); Klucel HF™ (hydroxypropyl cellulose, molecular weight 1,150,000); Klucel NF™ (hydroxypropyl cellulose); Klucel MF (hydroxypropyl cellulose, molecular weight 850,000), Klucel GF (hydroxypropyl cellulose, molecular weight 370,000), Klucel JF (hydroxypropyl cellulose, molecular weight 140,000), Klucel LF (hydroxypropyl cellulose, molecular weight 95,000), Klucel EF (hydroxypropyl cellulose, molecular weight 80,000), and Natrosol 250HX (hydroxyethylcellulose) each available from Hercules Incorporated, Wilmington, DE (supplied by Aqualon).

Other examples of commercially available swellable polymers include L-HPC Grade 11™ (Low Substituted hydroxypropyl cellulose), available from Shin-Etsu Chemical Co., Ltd., via Biddle Sawyer Corp., New York, NY;

Other examples of commercially available swellable polymers include Primellose™ (Croscarmellose Sodium); Monkey 4™ (Sodium Starch Glycolate); each (available from Avebe, via Generichem Corporation, Totowa, NJ);

Other examples of commercially available swellable polymers include Carbopol 974P™(polyacrylic acid cross-linked with polyalkenyl ethers or divinyl glycol); Carbopol 934P (polyacrylic acid); Carbopol 971P (polyacrylic acid cross-linked with polyalkenyl ethers or divinyl glycol); each available from Noveon, Inc., Cleveland, OH.

Other examples of commercially available swellable polymers include polyvinyl alcohols available from DuPont, such as Elvanol^{®} 71-30, Elvanol^{®} 85-30, Elvanol^{®} 50-42, and Elvanol^{®} HV.

Addition of hydro-attractants can improve the swelling properties of a gastro-retentive dosage form significantly, and hence can constitute a swellable polymer. Examples of hydro-attractants which can be incorporated into pharmaceutical compositions of the present invention include crosslinked poly(acrylic acid), crosslinked poly(vinyl pyrrolidone), microcrystalline cellulose, crosslinked carboxymethyl cellulose, starch granules, sodium carboxymethyl starch, alginates, low substituted hydroxypropyl cellulose (L-HPC, 10-13% substitution by weight, Shin-Etsu Chemical Company, Ltd, distributed by Biddle Sawyer), Croscarmellose Sodium (Primellose) (Avebe, distributed by Generichem), Sodium Starch Glycolate (Avebe, distributed by Generichem) sodium phosphates, such as disodium phosphate, sodium chloride, sodium citrate, sodium acetate, succinic acid, fumaric acid, tartaric acid, tannic acid, sugars (eg. manitol, sucrose, lactose, fructose, sorbital) and natural amino acids.

### Release Controlling Polymers

In one embodiment of the present invention, a pharmaceutical composition comprises an active agent, a delivery agent and at least one release controlling polymer. The pharmaceutical composition may contain, for example, 1 to 60% by weight of the release controlling polymer.

In a further embodiment of the present invention, the pharmaceutical composition comprises an active agent, a delivery agent, a swellable polymer and at least one release controlling polymer. In one embodiment of the present invention, the release controlling polymer allows the pharmaceutical composition to be released at its surface. In such embodiments, the dissolution of the tablet or dosage form at the surface reduces the increase in volume caused by the swellable polymer.

Examples of release controlling polymers include, for example, poly(ethylene oxide), poly(acrylic acid), polyvinyl alcohol, alginate, chitosan, polyvinylpyrrolidone, cellulose polymers and polysaccharides.

Release controlling polymers are often selected from the same class as swellable polymers, but have lower viscosity and molecular weights. Some polymers both expand the pharmaceutical composition, and control the release of the composition. Accordingly, a polymer may be both a swellable polymer and a release controlling polymer.

Commercially available release controlling polymers include, for example, PolyOX WSR N750 ((Poly(ethylene oxide), molecular weight 300,000), PolyOX WSR N80 ((Poly(ethylene oxide), molecular weight 200,000), and PolyOX WSR N10 (Poly(ethylene oxide), molecular weight 100,000), Methocel A15-LV, Methocel A4CP, Methocel A15CP, and Cellosize WP, and Cellosize QP available from Dow Chemical Company, Midland MI.

Other commercially available release controlling polymers include, for example, Natrosol 250 (hydroxyethylcellulose), Klucel JF, Klucel LF, and Klucel EF available from Hercules Incorporated, Wilmington, DE (supplied by Aqualon); Ptotosan UP CL/G, Pronova UP LVG , Pronova UP MVG, Pronova UP MVM, and Pronova UP LVM, available from FMC Biopolymer, Philadelphia, PA; Gohsenol N, Gohsenol A, Gohsenol G, Gohsenol K available from Nippon Gohsei, Osaka, Japan; Kollidon F, available from BASF Corp., Florham Park, NJ.

### Mucoadhesives

In embodiments of the present invention, the pharmaceutical composition includes a mucoadhesive. The mucoadhesive facilitates retention in the stomach by binding to the mucosal surface of the stomach, or by association with the mucosal coat.

Examples of mucoadhesives include a polyacrylic acid or polyacrylate optionally cross-linked with allyl sucrose, allyl ethers of sucrose, allylpentaerythritol, pentaerythritol or divinyl glycol; a carboxylvinyl polymer; a polyvinyl pyrrolidone (PVP); polyvinyl alcohol; sodium carboxymethylcellulose (CMC); a dextran polymer; a copolymer of polymethyl vinyl ether and maleic anhydride; hydroxymethylcellulose; methylcellulose; a tragacanth; an alginic acid; gelatin; gum arabic; and a polysaccharide optionally interrupted with a ß-(1-4)-linked D-glucosamine unit and/or a N-acetyl-D-glucosamine unit, and mixtures thereof.

In one embodiment the mucoadhesive is Carbopol^{®} 934 P. In an application of this embodiment 5% by weight of Carbopol^{®} 934 P is added to a SNAC/Heparin composition and tableted.

In an alternative embodiment, the mucoadhesive is chitosan. In an application of this embodiment, 5% of chitosan is added to a SNAC/Heparin composition and tableted.

### Dosage form design

The swelling rate, swollen size and the mechanical strength of the pharmaceutical formulation are factors to be considered in the dosage form design. With regard to size, the diameter of the pylorus varies between individuals from about 1 to about 4 cm, averaging about 2 cm. The mean resting diameter in humans was reported to be 12.8 ± 7.0 mm. Non-disintegrated tablets that have sizes up to 13 mm in diameter are generally emptied from stomach. The larger the size, the longer the dosage forms retain. Tablets larger than 11 mm tended to emptied only during IMMC. In embodiments of the present invention, the size of the pharmaceutical composition was set to reach 2 - 2.5 cm before the IMMC in those patents.

With regard to the swelling rate/time, embodiments that do not include swellable polymers remain in the stomach on average for about 1 to 3 hours, depending on the initial size of the dosage form. There is a high probability that the expanding form could pass the pylorus before reaching the sufficient size to be retained. Thus, it is preferred to have fast initial swelling times, preferably swelling within 30 - 60 minutes.

Finally, with regard to the mechanical strength, the swollen dosage form should be rigid enough to be retained in the stomach before the active agent-drug delivery agent complex is completely released.

Because of the dramatic difference in solubility in gastric fluid between of various active agents and delivery agents of the present invention, it is often not possible to achieve the simultaneous release of the drug and the carrier through a diffusion mechanism upon which the GRDDS in the prior art is based. Therefore, embodiments of the present invention include release controlling polymer which facilitates surface erosion.

In one embodiment, the swellable polymer was PolyOX WSR 303, the release controlling polymer was PolyOX WSR N80, the active agent was Heparin, and the delivery agent was the sodium salt of SNAC.

### Selection of the swelling polymer

The Swelling polymer plays a key role in the expandable GRDDS. Numerous polymers can swell to a large volume have been reported. However, those that were approved for pharmaceutical use are limited. There are mainly three categories: (1) poly(ethylene oxide) series, (2) polysaccharide series and (3) poly(acrylic acid) series. The most frequently used pharmaceutical polymers for expandable GRDDS were poly(ethylene oxide) series (Alza, DepoMed) manufactured by Dow chemicals, such as PolyOX 303™ (M = 7,000K), PolyOX N12K™ (M = IⱼOOOK) etc., and cellulose series (Teva, DepoMed) such as Methocel K1 5PM™, Methocel F4M™, MethoceI E4M™ manufactured by Dow and BClucel HF (M = 1,150K) provided by Hercules.

### End Use Pharmaceutical Applictions

The present invention provides compositions for use in the treatment or prevention of a disease or for achieving a desired physiological effect in an animal. Preferably, an effective amount of the composition for the treatment or prevention of the desired disease or for achieving the desired physiological effect is administered. Specific indications for active agents can be found in the Physicians' Desk Reference (58^{th} Ed., 2004, Medical Economics Company, Inc., Montvale, NJ), and both of which are incorporated by reference. Examples of diseases and physiological effects which can be treated or achieved by administering a pharmaceutical composition of the present invention are set forth below:

| **Active Agent** | **Disease and Physiological Effect** |
|---|---|
| Heparin | Thrombosis; prevention of blood coagulation |

Following administration, the active agent present in the composition or dosage unit form is taken up into the circulation. The bioavailability of the agent can be readily assessed by measuring a known pharmacological activity in blood, e.g. an increase in blood clotting time caused by heparin, or a decrease in circulating calcium levels caused by calcitonin. Alternately, the circulating levels of the active agent itself can be measured directly.

### Examples

The following examples illustrate the invention. All parts are given by weight unless otherwise indicated.

### Example 1: Bi-lavered caplets /tablets

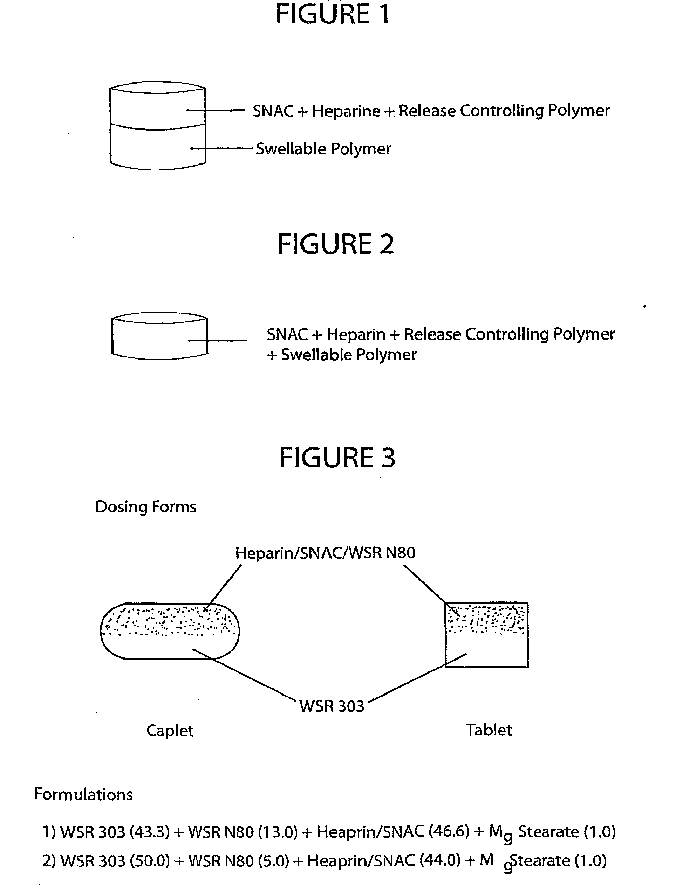

A bi-layered caplet is prepared having a physical blend of SNAC, heparin, and release controlling polymer in one layer, and a swellable polymer in another layer. This is shown in Figure 1.

In this embodiment, Heparin/SNAC release is achieved through surface erosion of the release controlling polymer. To keep heparin and SNAC in close proximity at the molecular level, heparin and SNAC are co-dried and powdered before blending with the release controlling polymer. The swellable polymer is set in another layer, and is responsible for the swelling of the dosage forms. These two layers were attached to each other via the physical bonds formed during compression.

### Reference Example 2: Matrix caplets/tablets

A one layered caplet is prepared having co-dried SNAC/heparin, a release controlling polymer, and a swellable polymer. This is shown in Figure 2.

The components compressed into a tablet or caplet. Heparin/SNAC release is achieved through surface erosion of the release controlling polymer.

### Reference Example 3: Preparation of Components of Pharmaceutical Formulations Co-dried heparin/SNAC

Heparin (1.2508 g) and SNAC (3.2485 g) were dissolved in 25 ml of deionized water. The solution was dried with nitrogen flow at room temperature overnight. The obtained solid cake was further dried under vacuum for 24 hours. The solid was then milled and screened through a 60-mesh sieve. The co-dried heparin/SNAC powder contained 13.1 wt% water. It was kept in desiccant for further use.

### Simulated gastric fluid (SGF)

Sodium chloride (2.0 g) was dissolved in 800 ml of deionized water. The solution was adjusted to pH 1.2 with hydrochloric acid (37%) and then diluted to 1000 ml with deionized water.

### Simulated intestinal fluid (SIF)

Monobasic potassium phosphate (6.8 g) was dissolved in 250 mi of deionized water. 77 ml of 0.2 N sodium hydroxide and 500 ml of deionized water was added to the solution. The solution was adjusted to pH 6.8 with either 0.2N sodium hydroxide or 0.2N hydrochloric acid and then diluted to 1000 ml with deionized water.

### HPLC analysis of heparin and SNAC

Heparin concentration was measured with a SEC column (PL aquagel-OH 30 8 um, 300 x 7.5 mm), mobile phases: 0.2M sodium sulfate (pH 5). Detector: UV206 nm.

SNAC concentration was measured with a Phenomenex column (Luna 5u C18, 75 x 4.6 mm, 5 Micro), mobile phases: A, 0.1% TFA in water; B, 0.1% TFA in acetonitrile; Detector: UV280 nm

### Example 4 - Swelling tests

A series swelling tests were performed on various swellable polymer/hydroattractant combinations containing either PolyOX 308™ or Methocel K15PM™.

The swellable polymer ingredient and Mg stearate (1 wt%) were manually blended. The blend was compressed into a flat-faced plain tablet on a Carver press at a pressure of 1000 psi. The tablets thus prepared ranged in weight 1000 ± 50 mg with a diameter of 13 ± 0.05 mm and a height / thickness of 6.5 ± 0.25 mm.

The tablet was added to 40 ml of modified SGF (without pepsin) in a 50ml beaker, which was maintained at 37 ± 2 °C. The tablet was taken out at a given time with a tweezers and gentle blotted dry with a kimwiper. The diameter (D) and the thickness (T) were measured with a calibrated electronic caliper. The volume was calculated based on the D and T. The strength of the swollen tablet was assessed qualitatively with the tweezers.

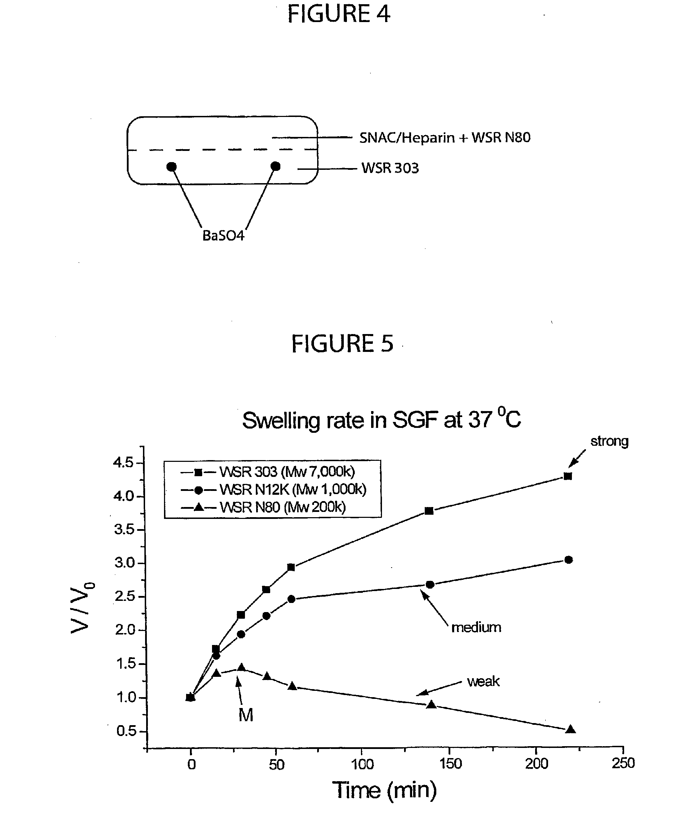

Figure 5 shows the swelling profiles of polyethylene oxide of three different molecular weights in SGF at 37 °C. Higher molecular weights provided higher Initial swelling rates, swelling volumes and mechanic strength. PolyOX WSR 303 which has an average molecular weight of 7,000K gave best swelling performance. Its volume doubled in ~ 30 minutes and kept increasing up to 4 - 4.5 times in ~ 3.5 hours (maximum test time) without comprising its structural integrity. It is believed that this formulation can be retained in the stomach for much longer that the 4 hours tested. For PolyOX WSR N12K with an average molecular weight of 1,000K, the swelling was significantly slower reaching ~ 3 times the baseline size in about 3.5 hours. It started to lose its mechanic strength at 1 - 1.5 hours. PolyOX WSR N80 which has a much lower molecular weight of 200K exhibited a unique swelling profile. It swelled to ~ 1.5 times in ~ 30 minutes with weakened mechanic strength, then started to lose the volume due to dissolution. Most of the polymer dissolved at the end of the test (3.5 hours) and the volume of the rest of the swollen tablet was about half of the original. This unique swelling property is useful in controlling the release rate of the drug and the carrier through surface erosion.

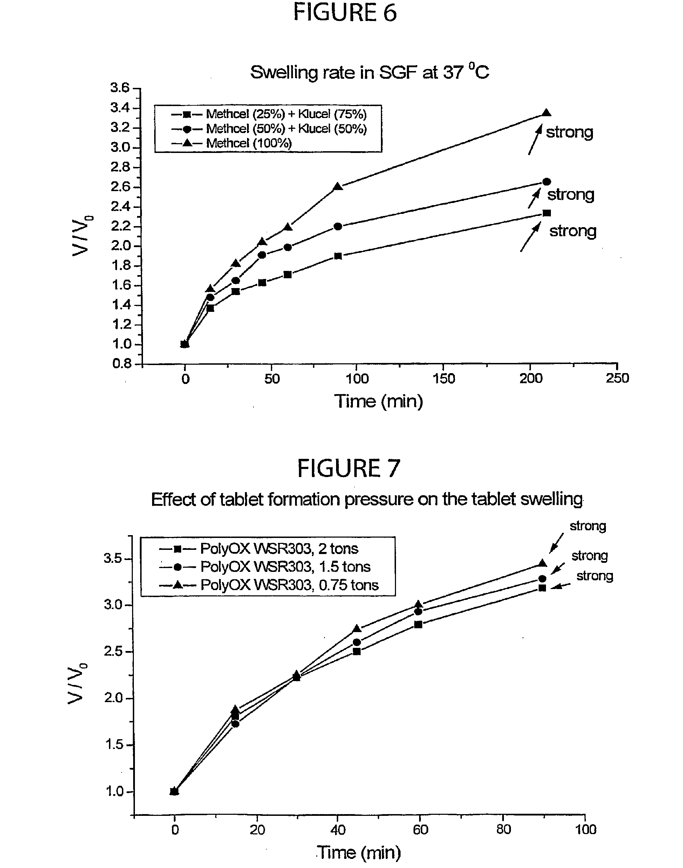

Effects of hydroattractants on the swelling of PolyOXWSR 303 were tested under same conditions. As shown in Table 1, addition of 50% of various hydroattractants including L-HPC, Primejel (Starch Glycolate), Primellose (croscarmellose sodium), Klucel (hydroxypropylcellulose) or their combinations did not improve the swelling of the polymer. Same trend was observed for Methocel^{®} K15PM (Methyl cellulose), as shown in Figure 6. Based on the result of the swelling test, it seems that Klucel did not improve the swelling performance of Methocel^{®}.

**Table 1 Swelling of polyOX WSR 303 tablets containing various hydro-attractants**

| | | | | | | |
|---|---|---|---|---|---|---|
| A --- WSR 303 (100%), B --- WSR 303 (509%) + L-HPC(50%), | | | | | | |
| C --- WSR 303 (50%) + Klucel HF (20%) + Primejel (30%); | | | | | | |
| D--- WSR 303 (50%) + Klucel HF (50%), E --- WSR 303 (50%) Primejel (50%) | | | | | | |
| F --- WSR 303 (50%) + Primellose (50%) | | | | | | |

| T | A | B | C | D | E | F |
|---|---|---|---|---|---|---|
| (Min) | V/V₀ Strength | V/V₀ Strength | V/V₀ Strength | V/V₀ Strength | V/V₀ Strength | V/V₀ Strength |
| 0 | 1 Strong | 1 Strong | 1 Strong | 1 Strong | 1 Strong | 1 Strong |
| 15 | 1.72 Strong | 1.81 Strong | 1.60 Strong | 1.69 Strong | 1.80 Strong | 1.73 Strong |
| 30 | 2.23 Strong | 2.1 Strong | 1.89 Strong | 1.90 Strong | 2.14 Strong | 1.96 Strong |
| 45 | 2.60 Strong | 2.5 Strong | 2.06 Strong | 2.10 Strong | 2.43 Strong | 2.31 Strong |
| 60 | 2.93 Strong | 2.82 Strong | 2.18 Strong | 2.20 Strong | 2.79 Strong | 2.51 Strong |
| 90 | | 3.1 medium | 2.23 Strong | 2.31 Strong | 3.05 Strong | 2.71 Strong |
| 150 | 3.8 Strong | (Erosion) | 2.73 Strong | 2.75 Strong | 4.42 Strong | 3.73 Strong |
| 19x60 | 8.46 Weak | Disintegrated | 6.2 Medium | 7.77 Weak | 9.9 Medium | 4.95 Weak |
| 24X60 | | | | | | |

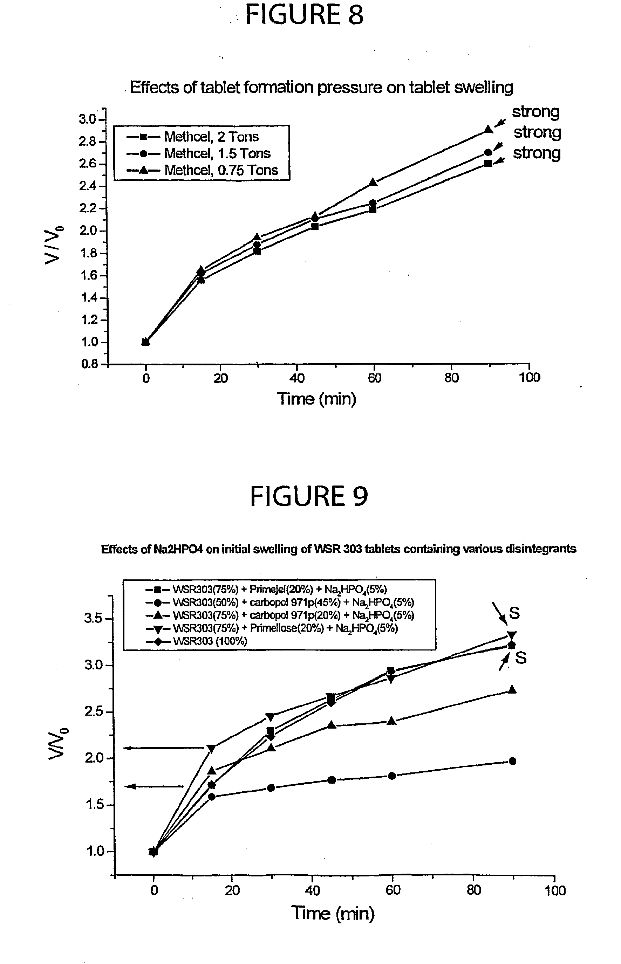

For both PolyOX WSR 303™and Methocel^{®} K15PM™ tablets, the effect of the compression pressure was not significant, as shown in Figures 7 and 8.

Carefully adjusting the ratio of Methocel^{®}/Klucel or superdisintegrant, such as Primejel or Primellose, can be effective to achieve fast initial swelling but generally with a compromise in mechanic strength. To solve this problems, ~ 4-5% tannic acid sometimes was added. Swelling test on these combinations was performed and the results were listed in Table 2.

**Table 2 Effects of tannic acid and pressure on swelling of Methocel^{®}-based tablets**

| Time H | A | | B | | C | | D | | E | | F | | G* | | | |
|---|---|---|---|---|---|---|---|---|---|---|---|---|---|---|---|---|
| (min) | V/V₀ | S | V/V₀ | S | V/V₀ | S | V/V₀ | S | V/V₀ | S | V/V₀ | S | V/V₀ | S | V/V₀ | S |
| 0 | 1 | S | 1 | S | 1 | S | 1 | S | 1 | S | 1 | S | 1 | S | | |
| 1 | S | | | | | | | | | | | | | | | |
| 15 | Swelled very fast, disintegrated | | | | | | 1.6 | S | 1.85 | S | Disintegrated | | 3.9 | W/S | | |
| 1.67 | S | | | | | | | | | | | | | | | |
| | | in 10 -15 min. | | | | | | | | | in 10 min | | | | | |
| 30 | | | | | | | 2.05 | S | 1.91 | S | | | 9.5 | W | 1.77 | S |
| 45 | | | | | | | 2.16 | S | 1.98 | S | | | 12.4 | W | 1.87 | S |
| 60 | | | | | | | 2.16 | S | 2.1 | S | | | Disintegrated | | 1.89 | S |

| | | | | | | | | | | | | | | | | |
|---|---|---|---|---|---|---|---|---|---|---|---|---|---|---|---|---|
| T = Thickness; D = Diameter; S = Strong; M = Medium; W = Weak • The V/V₀ is estimated value, the shape was irregular. A: Methocel^{®} (23.8%) + Primejel (71.4%) + Tannic acid (4.8%), 2 tons B: Methocel^{®} (23.8%) + Primellose (71.4%) + Tannic acid (4.8%), 2 tons C: Methocel^{®} (26.7%) + Klucel (16.0%) + Primejel (53.3%) + Tannic acid (4%), 2 tons D: Methocel^{®} (15.9%) + Klucel (47.6%) + Primejel (31.7%) + Tannic acid (4.8%), 2 tons E: Methocel^{®} (30%) + Klucel (15%) + Primejel (55%), 0.75 tons F: Methocel^{®} (30%) + Klucel (15%) + Primejel (50.2%) + Tannic acid (4.8%), 0.75 tons G: Methocel^{®} (15.9%) + Klucel (47.6%) + Primejel (31.7%) + Tannic acid (4.8%), 0.75 tons H: Methocel^{®} (31.8%) + Klucel (31.7%) + Primejel (31.7%) + Tannic acid (4.8%), 0.75 tons | | | | | | | | | | | | | | | | |

As shown in the table, most combinations (formulations A, B, C, F and G) swelled very fast and lost their integrity dramatically (10 - 15 min) due to the presence of the superdisintegrants. Addition of tannic acid of 4-5% could not prevent the tablets from disintegration. Carefully adjusting the ratio could help maintain integrity, however, the swelling was compromised (formulations D and H). Compression had a significant effect on the swelling (formulation D and G). The lower the compression pressure, the faster the swelling associated with quicker loss in mechanic strength.

The effect of inorganic phosphate salt such as monosodium phosphate on the swelling of the polymer tablet was also evaluated. As shown in Figure 9, different combination of PolyOX WSR 303™/primejel or primellose or carbopol all with 5% Na₂HPO₄ gave different initial swelling patterns. Addition of 20% primellose and 5% Na₂HPO₄ appeared to improve the initial swelling of Poly WSR 303™ tablet without causing a compromise in integrity. In 15 min, the tablet of this combination swelled to 2.2 times in comparison to ∼ 1.7 times for the PolyOX WSR 303 only.

As mentioned above, lower molecular weight PolyOX WSR N80™ could be used as an additive component to adjust the release rate of the drug and the carrier through surface erosion. A test was conducted to evaluate the effect of the presence of the polymer on the swelling of PolyOX WSR 303™ and Methocel^{®} K15PM. As shown in Tables 3 and 4, upon addition of up to 25% PolyOX WSR N80™, both the swelling profile and the mechanic strength of either PolyOX WSR 303™ or Methocel^{®} K15PM matrix tablets were well maintained. When the content of PolyOX WSR N80™ was over 50% both the initial swelling and the mechanic strength were compromised.

**Table 3 Swelling test of polyOX WSR303 / polyOX WSR N80 blends (SGF, 37 °C)**

| Time (min) | WSR 303 (250mg) | | | | WSR 303 (500mg) | | | | WSR 303 (750mg) | | | |
|---|---|---|---|---|---|---|---|---|---|---|---|---|
| | WSRN80 (750mg) | | | | WSR N80 (500mg) | | | | WSR N80 (250mg) | | | |
| | T | D | V/V₀ | Strength | T | D | V/V₀ | Strength | T | D | V/V₀ | Strength |
| 0 | 6.6 | 12.95 | 1 | S | 6.61 | 12.95 | 1 | S | 6.65 | 12.95 | 1 | S |
| 15 | 8.48 | 14.70 | 1.66 | S/M | 8.97 | 14.87 | 1.79 | S | 9.22 | 15.07 | 1.88 | S |
| 30 | 9.28 | 14.88 | 1.86 | M | 9.74 | 15.13 | 2.01 | S | 10.07 | 15.50 | 2.17 | S |
| 45 | 9.42 | 15.20 | 1.97 | M | 10.5 | 15.45 | 2.26 | S | 11.04 | 16.30 | 2.63 | S |
| 60 | 9.72 | 15.0 | 1.98 | M | 11.13 | 16.08 | 2.60 | S | 11.34 | 16.53 | 2.78 | S |
| 90 | 9.80 | 14.20 | 1.78 | M | 11.33 | 16.44 | 2.76 | S/M | 11.55 | 16.74 | 2.90 | S |
| 150 | 10.1 | 13.8 | 1.74 | W | 12.1 | 17.4 | 3.30 | M | 12.25 | 17.50 | 3.36 | S |
| 24x60 | Disintegrated | | | | Disintegrated | | | | Disintegrated | | | |

| | | | | | | | | | | | | |
|---|---|---|---|---|---|---|---|---|---|---|---|---|
| T = Thickness; D = Diameter; S = Strong; M = Medium; W = Weak | | | | | | | | | | | | |

**Table 4 Swelling test of Methocel^{®} / polyOX WSR N80 blends (SGF, 37 °C)**

| Time (min) | Methocel^{®} (250mg) WSR N80 (750mg) | | | | Methocel^{®} (500mg) WSR N80 (500mg) | | | | Methocel^{®} (750mg) WSR N80 (250mg) | | | |
|---|---|---|---|---|---|---|---|---|---|---|---|---|
| | T | D | V/V₀ | Strength | T | D | V/V₀ | Strength | T | D | V/V₀ | Strength |
| 0 | 6.48 | 12.95 | 1 | S | 6.45 | 12.99 | 1 | S | 5.99 | 12.95 | 1 | S |
| 15 | 8.1 | 13.6 | 1.38 | S | 7.90 | 13.55 | 1.33 | S | 8.21 | 15.07 | 1.60 | S |
| 30 | 8.3 | 13.8 | 1.45 | S/M | 8.95 | 14.26 | 1.67 | S | 0.05 | 15.50 | 1.87 | S |
| 45 | 8.4 | 13.8 | 1.47 | M | 9.50 | 14.56 | 1.85 | S/M | 9.78 | 16.30 | 2.17 | S |
| 60 | 8.5 | 14.2 | 1.58 | M/W | 9.85 | 14.85 | 2.0 | S/M | 10.82 | 16.53 | 2.45 | S |
| 90 | 8.2 | 14.03 | 1.49 | M/M | 10.10 | 15.7 | 2.29 | S/M | 11.42 | 16.74 | 2.63 | S |
| 150 | 8.0 | 13.5 | 1.34 | W | 9.89 | 15.8 | 2.27 | M/W | 12.92 | 17.50 | 3.18 | S |
| 210 | 7.0 | 12.3 | 0.97 | W | 10.5 | 13.8 | 1.81 | W | 13.72 | 16.09 | 3.52 | S |
| 24x60 | Disintegrated | | | | Disintegrated | | | | Disintegrated | | | |

| | | | | | | | | | | | | |
|---|---|---|---|---|---|---|---|---|---|---|---|---|
| T = Thickness; D = Diameter; S = Strong; M = Medium; W = Weak | | | | | | | | | | | | |

In summary, both PolyOX WSR 303 and Methocel^{®} K15PM showed good swelling properties. In terms of initial swelling rate/volume and mechanic strength, PolyOX WSR 303 appeared to provide better results than Methocel® K15PM. Addition of hydroattractants and/ or superdisintegrants caused significant changes in the polymer swelling. Lower molecular weight polyethylene oxide, PolyOX WSR N80, exhibited a unique swelling property. The volume reached the maximum in ∼ 30 min followed by significant drop in the volume due to dissolution. The swelling property can be used to adjust the release rate of the drug/carrier through surface erosion. With the consideration of their structural similarity, PolyOX WSR 303™ and PolyOX WSR N80™ were selected as the swellable polymer and release controlling polymer, respectively, for the drug/carrier loaded dosage forms that were used for the in vitro and in vivo studies.

### Heparin/SNAC loaded tablet

To test the swelling of proposed GRDDS dosage forms, heparin/SNAC loaded matrix tablet and bi-layered tablet were made with such a formulation in which heparin to SNAC ratio was 3:8 (w/w) and (heparin + SNAC) to (WSR 303 + WSR N80) ratio was ∼ 4:5 (w/w). For the tablet containing WSR N80, the ratio of WSR N80 to WSR 303 was 1:3. The tablets weighed 940-960 mg. The matrix tablet was made from a physical blend of all the ingredients (see Figure 2); the bi-layered tablet contained the swellable polymer in one layer and the remaining ingredients in the other (see Figure 1).

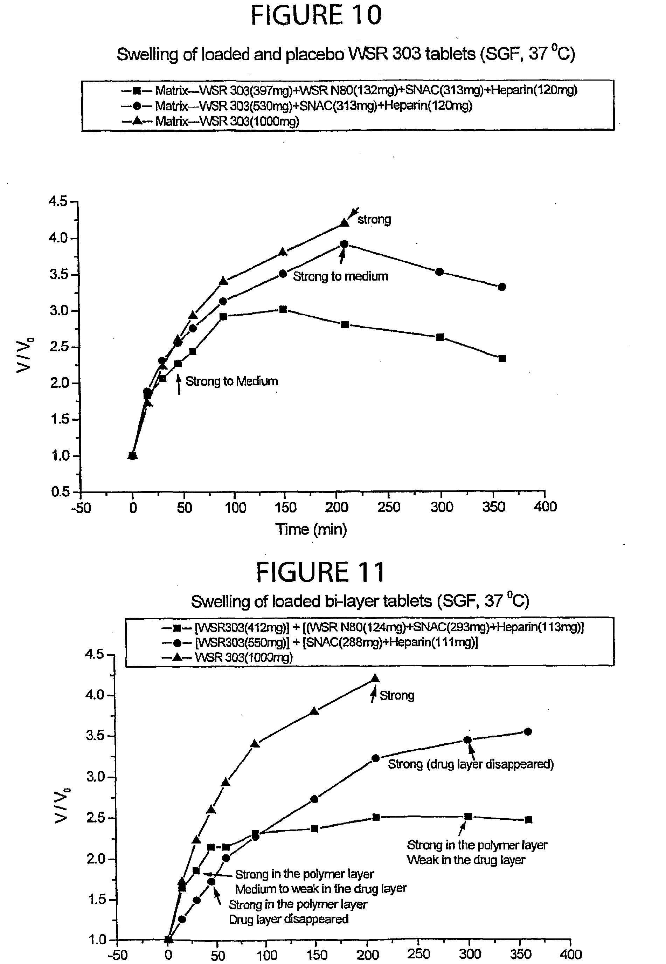

The swelling properties of these tablets were tested with same method set forth above. As shown in Figure 10, the initial swelling of the loaded matrix tablet with or without the release controlling polymer (WSR N80) were very comparable to the placebo tablet (tablet containing only WSR 303), reaching 2 - 2.2 times in ∼ 30 min. with strong mechanic strength. Because of surface erosion caused mainly by dissolution of WSR N80, the matrix tablet containing WSR N80 started to lose the mechanic strength at ∼ 30-45 min, which was consistent with that reflected in the swelling profile of WSR N80 (See Figure 5). The matrix tablet that did not contain WSR N80 did not lose the mechanic strength until 3.5 h where the released heparin/SNAC became significant. Due to the competition of volume increase from polymer swelling and volume decrease from WSR N80 dissolution and release of heparin/SNAC, a maximum swollen volume was observed at 2 -2.5 h for the matrix tablet with the release controlling polymer and at 3-4 h for the matrix tablet without the release controlling polymer. As reflected in the swelling profiles, the swollen volume might correlate to the amount of the swellable polymer in the tablets from 0.5 h to 1.5 h since the volume increase dominated the competition.

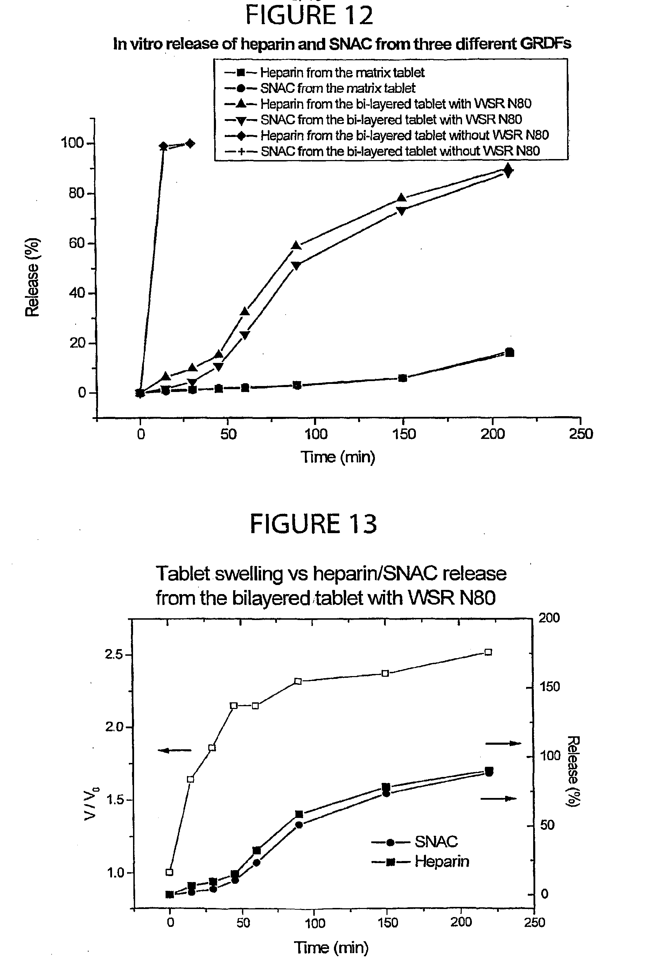

Compared to the loaded matrix tablet, the initial swelling of the loaded bi-layered tablet was significant slower (Figure 11) due to the unique design of tablet from which the release of heparin/SNAC was much faster and the surface volume used for swelling was significant smaller. As discussed below, when the active agent/delivery agent layer did not contain the release controlling polymer, i.e., WSR N80, the release completed in ∼ 30-45 min compared to 4-5 h for layers that contained the release controlling polymer. (See Figure 12). For the bi-layered tablet with the release controlling polymer polymer, the volume reached maximum plateau of 2.2 times at 45 - 60 min that lasted for ∼ 4 h. During this time period, the volume increase from swelling balanced the volume decrease from the erosion of the drug/carrier layer.

### Example 5 -- In vitro release / dissolution

At least three processes are involved in heparin / SNAC absorption when the gastric retention dosage form is administered --- release of heparin / SNAC in the stomach (in precipitate or in solution), dissolution of heparin / SNAC in the stomach and dissolution of heparin / SNAC in the intestine. All three process were tested in simulated fluid at 37 °C.

Tablets were compressed under 1000 psi (1.5 tons), weighing 1000 ± 50mg with a diameter of 13 ± 0.05 mm and a height / thickness of 6.5 ± 0.25 mm.

### Release Of Heparin/SNAC In The Stomach

The in vitro release experiments were out carried in SGF (40 ml / tablet) at 37 ± 2 °C with gentle stirring. The tablet was taken out the flask at the given time point. The SGF media containing released SNAC/heparin precipitate/solution was adjusted to pH 9-10 with 5N NaOH, and then diluted to 50 or 100 ml. The concentrations of both heparin and SNAC were measured by HPLC.

Three dosage forms were tested. The first dosage form was the matrix tablet shown in Figure 2. The second dosage form was the bi-layer tablet shown in Figure 1, containing 13 wt% release controlling polymer (WSR N80). The third dosage form was the bi-layer dosage form of Figure 1, but without the release controlling polymer (WSR N80).

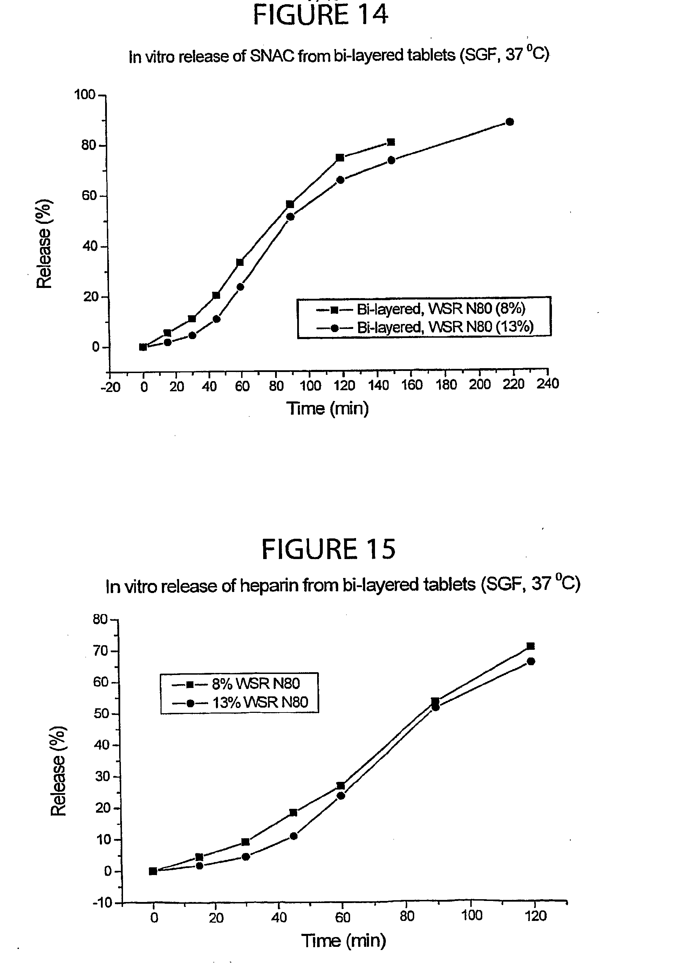

As shown in Figure 12, simultaneous release of heparin and SNAC were achieved from all the three dosage forms through a surface erosion process. The release rate could be adjusted by adjusting the amount of the release controlling polymer (see Figures 14-15 discussed below). An immediate release for the drug/carrier was observed for the bi-layered tablet without the release controlling polymer, whereas in the presence of 13% WSR N80, a sustained release of the drug/carrier was achieved due to gradual erosion of the drug/carrier layer.

Tablet swelling and heparin/SNAC release were correlated for the bi-layered tablet with WSR N80. The results are shown in Figure 13.

The slightly S-shaped release profile for the bi-layered tablet probably reflects the swelling effects on surface erosion. The surface area increases with increase in the volume of the tablet causing increase in surface erosion and thus the release rate. Most of the drug and the carrier were released after 1.5 h, thus decreasing the release rate.

In the case of the matrix tablet containing 13% of WSR N80, the release controlling polymer polymer was homogeneously distributed throughout whole tablet. The surface erosion process became much slower causing dramatic decrease in the release rate compared to the bi-layered tablet. The release rate became more significant after 1.5 h with the increase in the swelling volume.

The release rate of the active agent (heparin) and the delivery agent (SNAC) from the bi-layered tablet can be adjusted by varying the amount of the release controlling polymer polymer. As shown in Figures 14 and 15, the release rate increased with the decrease in the amount of WSR N80 in the drug layer.

### Dissolution Of Heparin /SNAC In The Stomach

Heparin and SNAC have very different solubility in acidic water at 37 °C, > 500mg/ml for heparin and < 0.1 mg/ml for SNAC (free acid form). Thus, after released from the tablet, almost all SNAC should exist in the SGF as precipitate whereas heparin should be in SGF solution.

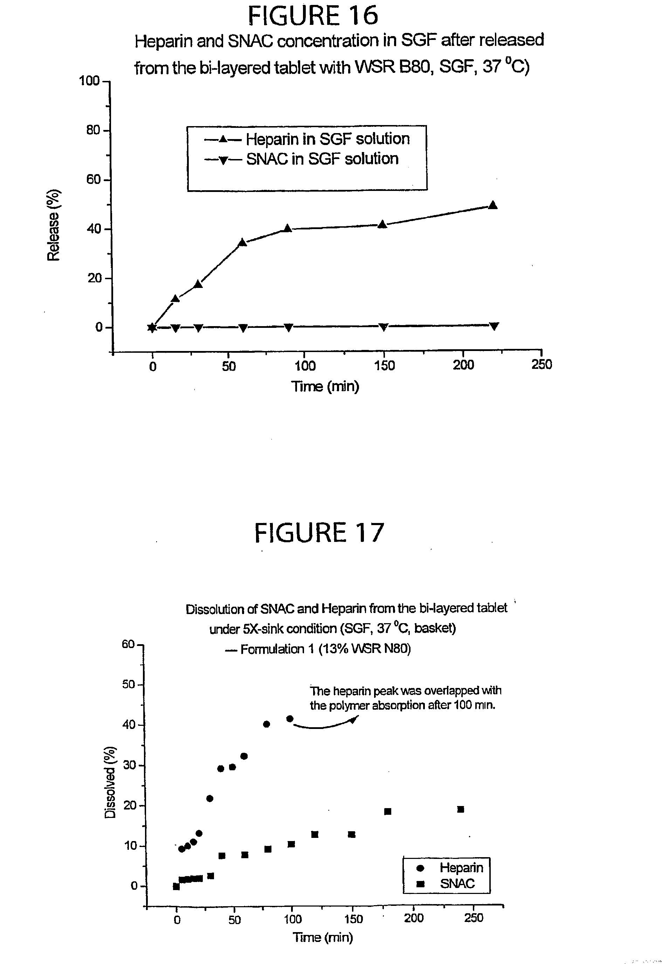

Figure 16 shows the release profiles of heparin and SNAC from the same bi-layered tablet containing 13% WSR N80, based on the solution concentrations of the two components in the acidic SGF. As expected, there was very little amount of SNAC (<0.1%) measured in the SGF solution whereas the heparin amount in the solution was very significant.

In the first 60 min, the heparin amount in the solution was comparable to the total amount of heparin released from the tablet (∼ 30%).

### Dissolution of heparin and SNAC in SGF under 5X sink condition

Sink conditions refer to the excess solubilizing capacity of the dissolution medium. 5X sink condition means five times the volume needed to completely dissolve the material in terms of the solubility. For this purpose, SNAC solubility in SGF at 37 °C was determined with HPLC, to be 0.07 mg/ml. The experiment was carried out in SGF at 37 °C on the Hanson SR 8-Plus system (available from Hanson Research, Chatsworth CA) at 75 rpm. A 50-mg bi-layered tablet, containing 12.84 mg of SNAC and 4.82 mg of heparin, with a diameter 7.1 mm and 950 ml of SGF was used.

The dissolved SNAC and heparin were measured by HPLC. For SNAC, large injection volume, 1 ml, was applied; for heparin, large sampling volume, 8 ml, was taken and then concentrated to 0.25 ml.

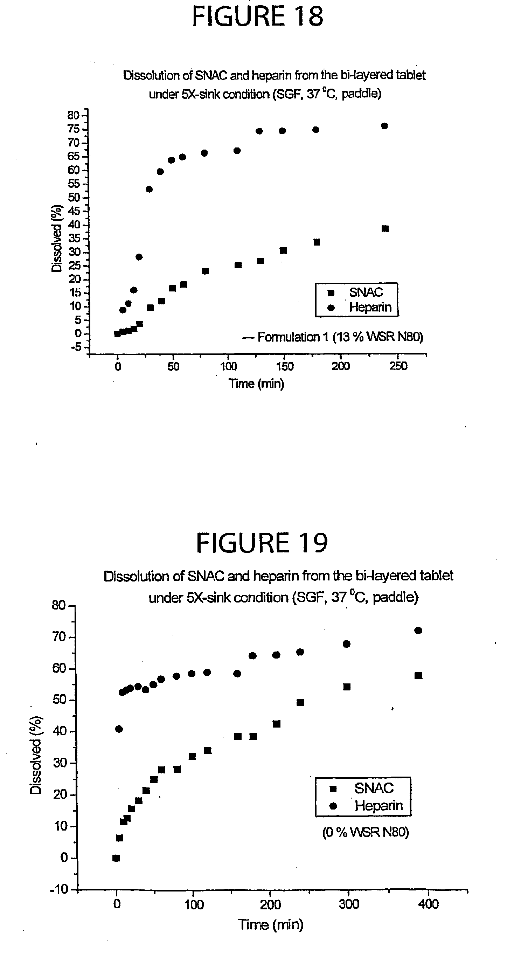

The experiment was performed in SGF at 37 °C using either a rotating basket or a paddle device. The dissolution of both heparin and SNAC from the same bi-layered tablet was measured. The dissolution profiles for SNAC and heparin are shown in Figures 17 and 18. Under 5X - sink condition in SGF, the dissolution rate of heparin was faster than that of SNAC. Agitation causes significant influence or the dissolution rates, and the paddle device gave higher dissolution rate compared to rotating basket with the same agitation speed (75 rpm).

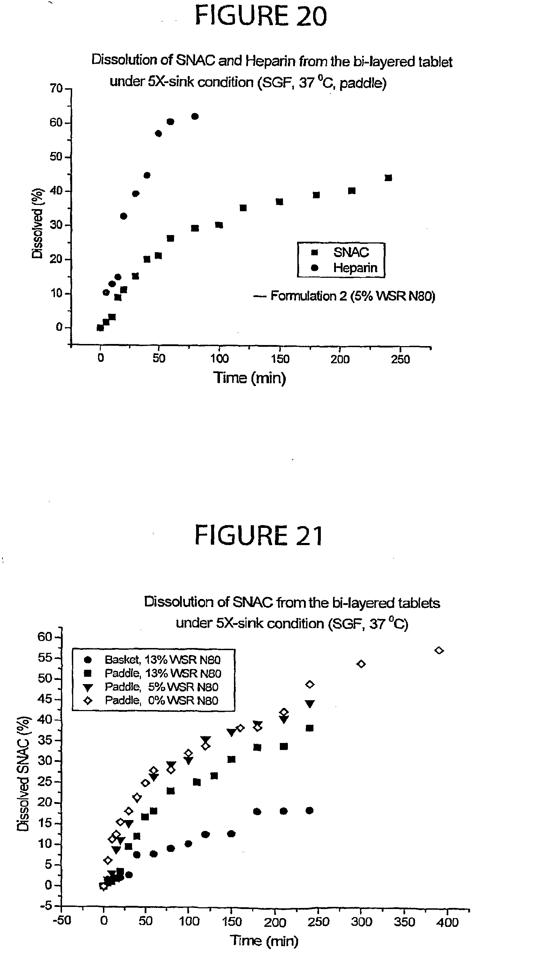

Similar bi-layered tablets containing 0% or 5% of WSR N80 were also tested under same conditions using the paddle device to study the effect of presence of the rate controlling polymer in the drug/carrier layer on the dissolution of the drug and the carrier. The results were shown in figures 19 & 20.

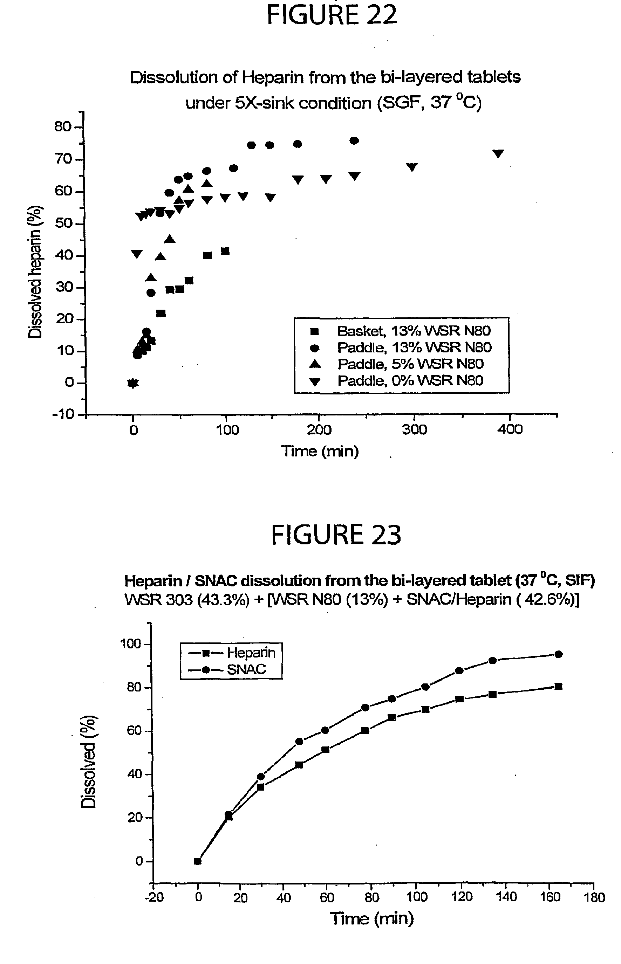

Figures 21 & 22 summarized the dissolution profiles of both SNAC and heparin. In all cases, heparin dissolution went faster than SNAC. The initial dissolution rate of both heparin and SNAC appeared to decrease with increase in the content of the release controlling polymer.

### SNAC/Heparin dissolution from the bi-layered tablet in SIF

The dissolution of both SNAC and heparin from the bi-layered tablet in Simulated Intestinal Fluid (SIF) was measured. The release of both heparin and SNAC from the bi-layered gastric retention dosage form was significantly prolonged (from ∼ 30 min. to ∼ 180 min.) due to the presence of the PolyOX WSR N80 in the drug/carrier layer (Figure 23).

Based on the profile, SNAC appears to dissolve faster than heparin. This is likely due to slower diffusion of macromolecular heparin through the polymer, as compared to the small molecular SNAC.

In summary, simultaneous release of heparin and SNAC from the gastric retention dosage form in SGF was achieved through the release controlling polymer-mediated surface erosion process. The release rates can be adjusted by varying the amount of the release controlling polymer (e.g. WSR N80). The active agent/delivery agent release is a hybrid process of swelling and surface erosion. Due to the presence of WSR N80 in the drug/carrier layer, the dissolution rates in SIF for both heparin and SNAC from the bi-layered tablet are significantly slower.

Heparin / SNAC absorption should be related to all the three process, among which heparin/SNAC release from the tablet in SGF may be the rate-limiting step.

### Example 6 - In vivo study in Rats

### Gastric retention and Heparin/SNAC absorption study in rats

Mini-tablets of the following four formulations were compressed for the tablet retention and heparin absorption study in rats (average body weight ∼ 350 g, n=3). The dose levels were 30 mg/kg of heparin and 80 mg/kg of SNAC. Group 1 and 2 were set up as two negative controls. In group 1, the formulation does not contain a release controlling polymer (WSR N80) in the active agent/delivery agent layer. In group 2, there was no swellable polymer (WSR 303) in the tablet. The formulations with the corresponding dosage forms were listed below:

Group 1, WSR 303 (45%) + Heparin/SNAC (54%) + Mg stearate (1%), bi-layered. WSR 303 was in one layer, and Heparin/SNAC was in the second layer.

Group 2, WSR N80 (19.5%) + Heparin/SNAC (79.5%) + Mg stearate (1%), one layer.

Group 3, WSR 303 (43.3%) + Heparin/SNAC (42.6%) + WSR N80 (13%) + Mg stearate (1%), bi-layered. WSR 303 was in one layer, and WSR N80 and Heparin/SNAC was in the second layer.

Group 4, WSR303 (50%) + Heparin/SNAC (44%) + WSR N80 (5%) + Mg stearate (1%), bi- layered. WSR 303 was in one layer, and WSR N80 and Heparin/SNAC was in the second layer.

### Preparation of the test articles

**Bi-layered** caplet for Group 1: 388 mg of WSR 303 and 4 mg of magnesium stearate were well mixed manually to form part A. 460 mg of the heparin/SNAC co-dried powder (KF, 17.12%) and 4 mg of magnesium stearate were well mixed to form part B. 23.2 mg of part B was weighed and added to the caplet die (size #2) as the first layer. 19.6 mg of the part A was then added to the top of the part B in the die as the second layer. The mixture was compressed under ∼ 1000 pound pressures on a Carver press to form a mini-caplet of 42.8 mg which contained 5.2 mg of heparin and 13.9 mg of SNAC. Based on two caplets per rat, the dose levels were 29.7 mg/kg of heparin and 79.4 mg/kg of SNAC for a 350 g rat.

**Bi-layered caplet for Group 2:** 460 mg of the heparin/SNAC co-dried powder (KF, 17.12%), 116 mg of WSR N80 and 4 mg of magnesium stearate were well mixed. 29.0 mg of the mixture was added to the caplet die (size #2) and compressed under ∼ 1000 pound pressures on a Carver press to form a mini-caplet which contained 5.2 mg of heparin and 13.9 mg of SNAC. Based on two caplets per rat, the dose levels were 29.7 mg/kg of heparin and 79.4 mg/kg of SNAC for a 350 g rat.

**Bi-layered caplet for Group 3:** 388 mg of WSR 303 and 4 mg of magnesium stearate were well mixed manually to form part A. 460 mg of the heparin/SNAC co-dried powder (KF, 17.12%), 116 mg of WSR N80 and 4 mg of magnesium stearate were well mixed to form part B. 29.0 mg of part B was weighed and added to the caplet die (size #2) as the first layer. 19.6 mg of the part A was then added to the top of the part B in the die as the second layer. The mixture was compressed under ∼ 1000 pound pressures on a Carver press to form a mini-caplet of 48.6 mg which contained 5.2 mg of heparin and 13.9 mg of SNAC. Based on two caplets per rat, the dose levels were 29.7 mg/kg of heparin and 79.4 mg/kg of SNAC for a 350 g rat.

**Bi-layered caplet for Group 4:** 388 mg of WSR 303 and 4 mg of magnesium stearate were well mixed manually to form part A. 460 mg of the heparin/SNAC co-dried powder (KF, 17.12%), 44 mg of WSR N80 and 4 mg of magnesium stearate were well mixed to form part B. 25.4 mg of part B was weighed and added to the caplet die (size #2) as the first layer. 22 mg of the part A was then added to the top of the part B in the die as the second layer. The mixture was compressed under ∼ 1000 pound pressures on a Carver press to form a mini-caplet of 47.4 mg which contained 5.2 mg of heparin and 13.9 mg of SNAC. Based on two caplets per rat, the dose levels were 29.7 mg/kg of heparin and 79.4 mg/kg of SNAC for a 350 g rat.

### Oral Gavage Procedures

Rat studies were carried out in Sprague Dawley rats (body weight was approximately 350 grams) by oral gavage administration. Rats were fasted for about 24 hours and anesthetized by intramuscular administration of ketamine (44 mg/kg) and thorazine (1.5 mg/kg). At pre-determined time intervals, blood samples were drawn from retro-orbital vessels and were appropriately prepared as either plasma or serum for glucose and insulin bioassays. The animal was sacrificed at the end of the experiment and rat GI mucosa was observed for any sign of local toxicity.

Based on polymer swelling, the GRDF was designed to provide a sustained release system that can deliver both the drug and the carrier at the same time at the same site (stomach). It is expected that the PK / PD profiles of the drug should be different from that of the non-sustained release dosage forms. Typically the Cmax may be lower whereas the action time range should be longer provided the gastric retention dosage form can retain in the stomach long enough for the active agent and delivery agent to complete release. Both rat and primate studies were performed to check if gastric retention and a sustained heparin PD profile can be achieved with the bi-layered tablet

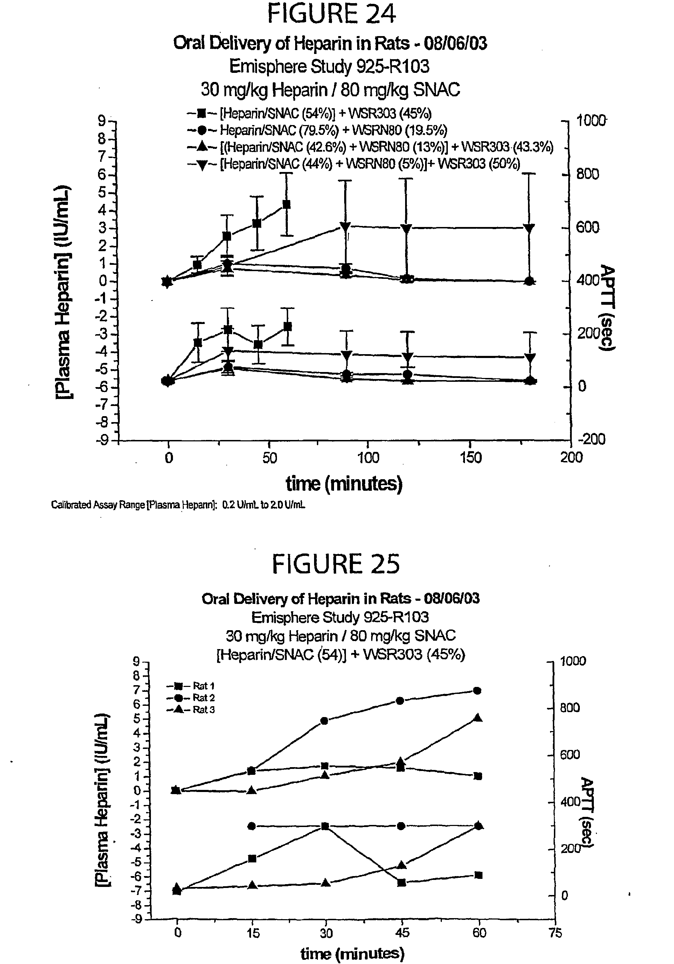

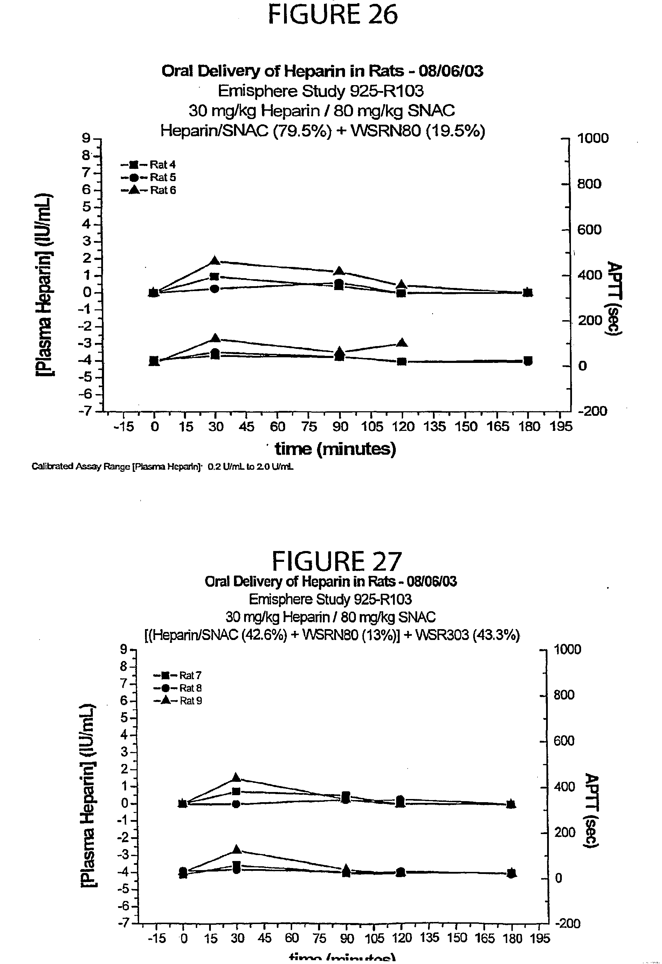

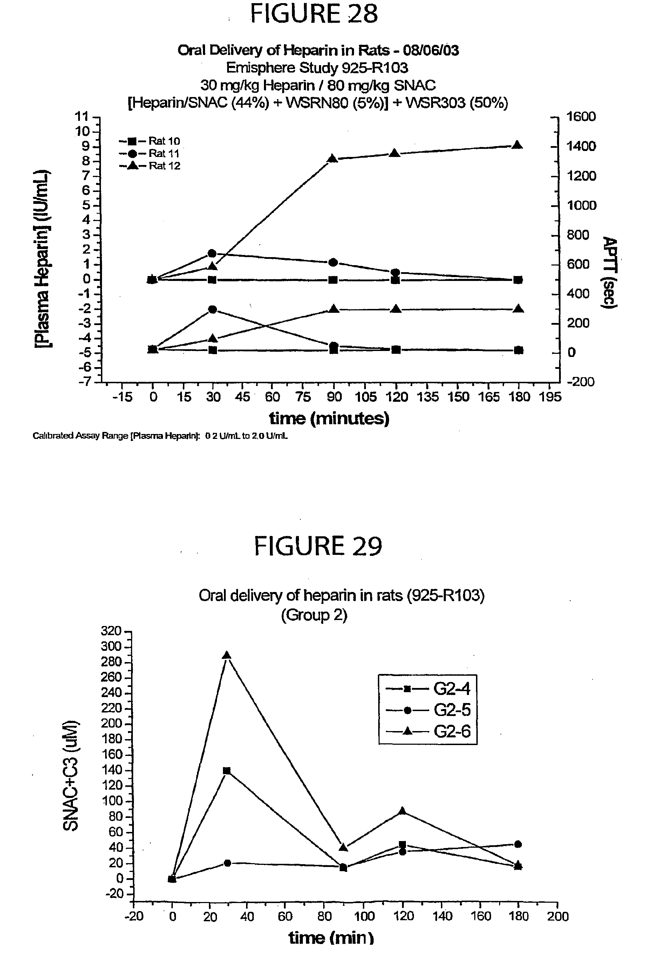

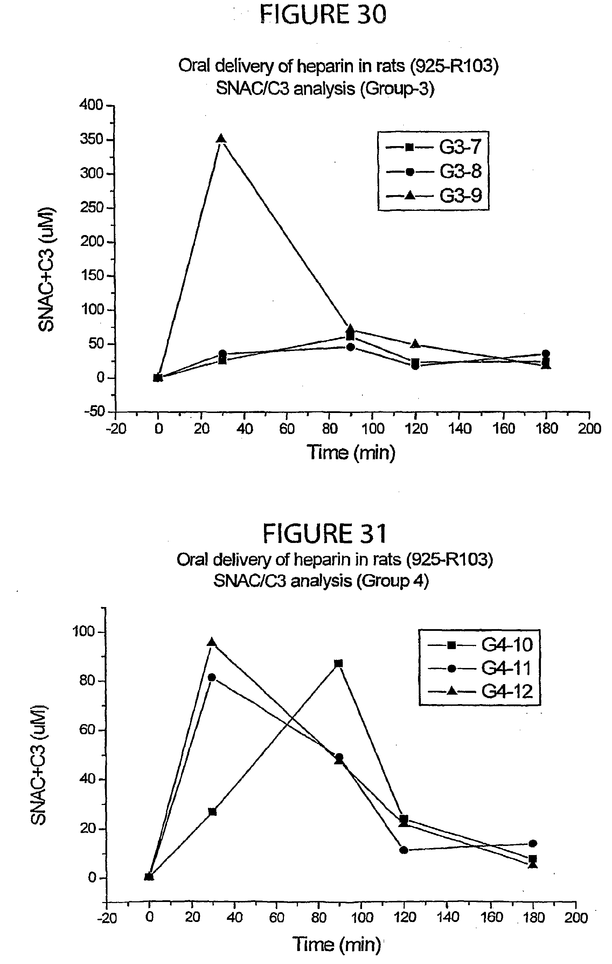

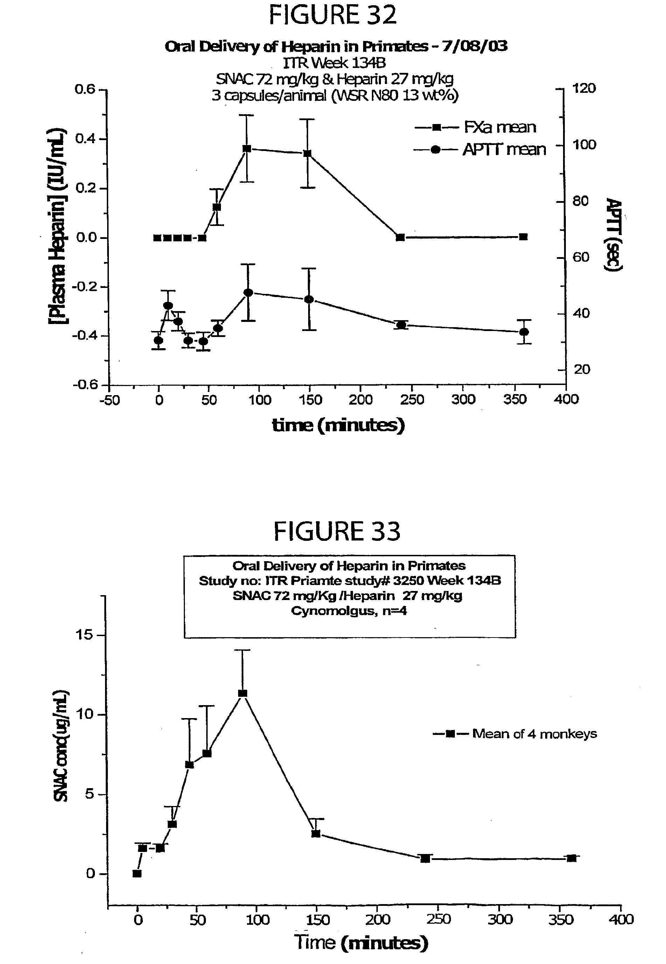

The mini-tablets, two for each animal, were oral administered to the rats and the blood sampling was taken at each pre-determined time points (0, 15, 30, 45 and 60 min for group 1; 0, 30, 90, 120 and 180 min for groups 2, 3 and 4) for heparin/SNAC absorption measuring. The gastric retentions of the tablets and the pH of the stomach fluid were checked through necropsy the end of the experiment. The results were listed in table 5. The heparin / SNAC absorption profiles of the four formulations from this rat study are shown in Figures 24 - 32 and were also summarized in the table 6. Figures 29 - 31 set forth SNAC/C3 concentrations, in which C3 is the 3-carbon metabolite of SNAC.

**Table 5 Gastric retention of tablets and the corresponding stomach pH in rats**

| Group / Rat# | Tablets found in the stomach | Stomach fluid pH |
|---|---|---|
| | | |
| G1 - 1 | 2 | 6 -7 |
| G1 - 2 | 1 (one disintegrated) | |
| G1 - 3 | 0 | |
| | | |
| G2 - 1 | 0 | 4-5 |
| G2 - 2 | 0 | 4-5 |
| G2 - 3 | 0 | 1-2 |
| | | |
| G3 - 1 | 1 (one disintegrated) | 4-5 |
| G3 - 2 | 2 | 5-6 |
| G3 - 3 | 1 (one disintegrated) | 4-5 |
| | | |
| G4 - 1 | 2 | 4-5 |
| G4 - 2 | 2 | 4-5 |
| G4 - 3 | 1 (one disintegrated) | 4-5 |

**Table 6 Absorption data of the small tablet rat experiment**

| Rat# | Heparin (Fxa, IU/ml) | | | | SNAC+C3 (uM) | | | |
|---|---|---|---|---|---|---|---|---|
| | Cmax | AUC | Tmax | Width | Cmax | AUC | Tmax | Width |
| G2 - 4 | 0.96 | 60.8 | 30 | 90 | 140.4 | 9444 | 30 | 90 |
| G2 - 5 | 0.6 | 38.3 | 90 | 90 | 44.9 | 4655 | 180 | 60 |
| G2 - 6 | 1.85 | 159.3 | 30 | 120 | 288.7 | 19270 | 30 | 90 |
| | | | | | | | | |
| G3 - 7 | 0.73 | 55.4 | 30 | 120 | 61.9 | 5756 | 90 | 90 |
| G3 - 8 | 0.26 | 23 | 120 | 90 | 46.2 | 5558 | 90 | 90 |
| G3 - 9 | 1.49 | 80.1 | 30 | 90 | 350.5 | 21752 | 30 | 90 |
| | | | | | | | | |
| G4 - 10 | --- | 0 | --- | --- | 87.2 | 6433 | 90 | 90 |
| G4 - 11 | 1.79 | 158 | 30 | 120 | 81.5 | 6800 | 30 | 120 |
| G4 - 12 | 8.55 (?) | 536 | 120 | 30 | 95.6 | 7575 | 30 | 90 |

As shown in table 5, two residual tablets were found in each animal of groups 1, 3 and 4, formulations which included the swellable polymer, polyOX WSR303. In the rats of group 2, which received the tablets containing no polyOX WSR303, no tablet retention was observed in the stomach. The stomach pH was mostly in the range of 4 - 6, slightly higher than expected due to the release of SNAC (a weak base).

Table 6 sets forth the heparin and SNAC absorption after oral administration of the bi-layered tablets containing the release controlling polymer, WSR N80, in the drug/carrier layer. (Thus, a sustained absorption profile was expected from them). As shown in the heparin absorption profiles, in terms of the shape, the heparin absorption does look like sustained for both the mean and the individual profiles. Typically, the Cmax is ∼ 0.6 to 1.8 whereas the action time is about 2 h. No delayed action time was observed.

### Example 7 - In vivo study in Primates

### Study design

Two groups of crossover studies were performed in 4 fasted Cynomolgus primates, 2 males and 2 females on the two dosage forms, caplet and tablet, of two related formulations: Study A (formulation 1, caplets); Study B (formulation 1, tablets); Study C (formulation 2, caplets); and Study D (formulation 2, tablets). Experiments Study A /Study B and Study C /Study D were crossover, respectively with 1 week washout period. The dose levels were 30mg/kg for heparin & 80 mg/kg for SNAC. The primate ID and body weights were
Study A --- 1M (5.1kg), 3M (5.2kg), 4F (6.7kg), 5F(6.4kg) --- av. 5.85kg
Study B --- 1M (5.5kg), 3M (5.4kg), 4F(7.2kg), 5F (6.2kg) --- av. 6.08kg
Study C --- 16M (4.4kg), 17M (3.7kg), 14F (3.2kg), 15F (3.1kg) --- av. 3.6kg
Study D --- 16M (4.3kg), 17M (3.8kg), 14F (3.1kg), 15F (3.2kg) --- av. 3.6kg

The blood samples were collected at pre-determined time points (0, 30, 60, 90 min. and 2.5h, 3h, 4h and 6h)and assayed on both heparin (APTT/FXa) and SNAC absorption. The dosage forms and the corresponding formulations tested in the studies were illustrated with dose volumes as follows:
Study A - Formulation 1 / caplets (3 caplets / monkey)
Study B - Formulation 1 / tablets (3 tablets / monkey)
Study C - Formulation 2 / caplets (2 caplets / monkey)
Study D - Formulation 2 / tablets (2 tablets / monkey)

Formulations 1 and 2 are set forth in Figure 3, in which the numbers denote wt%.

### Preparation procedures of the bi-layered caplets /caplets

**Bi-layered caplets for study A:** 3.92g of WSR 303 and 35.2 mg of magnesium stearate were well mixed manually to form part A. 4.82 g of the heparin/SNAC co-dried powder (KF, 19.06%), 1.172 g of WSR N80 and 52.8 mg of magnesium stearate were well mixed to give part B. 0.3022 g of part B was weighed and added to the caplet die (size #2) as the first layer. 0.1978 g of the part A was then added to the top of the part B in the die as the second layer. The mixture was compressed under 1.5 ton pressure on a Carver press to form a caplet of 500 mg which contained 53 mg of heparin and 141 mg of SNAC. Based on three caplets per primate, the dose levels were 27 mg/kg of heparin and 72 mg/kg of SNAC for a 5.85 kg primate or 30 mg/kg of heparin and 80 mg/kg of SNAC for a 5.25 kg primate.

**Bi-layered caplets for study C:** 4.5 g of WSR 303 and 50 mg of magnesium stearate were well mixed to form part A. 4.95 g of SNAC/heparin co-dried powder (KF, 19.06%), 0.45 g of WSR N80 and 50 mg of magnesium were well mixed to give part B. 0.2725 g of part B was weighed and added to the caplet die (size #2) as the first layer. 0.2275 g of the part A was then added to the top of the part B in the die as the second layer. The mixture was compressed under 1.5 ton pressures on a Carver press to form a caplet of 500 mg which contained 54.7 mg of heparin and 145.8 mg of SNAC. Based on two caplets per primate, the dose levels were 30.4 mg/kg of heparin and 81 mg/kg of SNAC for a 3.6 kg primate.

**Bi-layered tablets for study B:** 4.474 g of WSR 303 and 50 mg of magnesium stearate were well mixed to form part A. 5.306 g of SNAC/heparin co-dried powder (KF, 17.12%), 1.34 g of WSR N80 and 50 mg of magnesium were well mixed to give part B. 0.2751 g of part B was weighed and added to the tablet die (cylindrical, size #2 diameter) as the first layer. 0.1859 g of the part A was then added to the top of the part B in the die as the second layer. The mixture was compressed under 1.5 ton pressures on a Carver press to form a tablet of 461 mg which contained 49.3 mg of heparin and 131.4 mg of SNAC. Based on three tablets per primate, the dose levels were 25.3 mg/kg of heparin and 67.4 mg/kg of SNAC for a 5.85 kg primate or 28.2 mg/kg of heparin and 75.1 mg/kg of SNAC for a 5.25 kg primate.

**Bi-layered tablets for study D:** 4.5 g of WSR 303 and 45 mg of magnesium stearate were well mixed to form part A. 4.778 g of SNAC/heparin co-dried powder (KF, 17.12%), 0.45 g of WSR N80 and 45 mg of magnesium were well mixed to give part B. 0.2476 g of part B was weighed and added to the tablet die (cylindrical, size #2 diameter) as the first layer. 0.2134 g of the part A was then added to the top of the part B in the die as the second layer. The mixture was compressed under 1.5 ton pressures on a Carver press to form a tablet of 461 mg which contained 50.7 mg of heparin and 135.2 mg of SNAC. Based on two tablets per primate, the dose levels were 28.2 mg/kg of heparin and 75.1 mg/kg of SNAC for a 3.6 kg primate.

### Heparin / SNAC assay Procedures

Rat serum concentrations of insulin were determined using Heparin ELISA Test Kit (DSL Inc.). The limit of quantitation (LOQ) has been established at 12.5 µU/mL, with the calibrated linear range of the assay up to 250 µU/mL. Changes in blood glucose levels were measured using a glucometer.

### X-ray monitoring gastric retention study on the bi-layered caplets in primates

### Study design

To investigate the gastric retention of the bi-layered caplet (size #2) in primates with X-ray, barium sulfate bead were embedded into the swellable polymer layers as illustrated in Figure 4:

The study was carried out in four fasted Rhesus primates: MONKEY 1 (F, 5.0kg), MONKEY 2 (F, 5.2kg), MONKEY 3 (M, 4.4 kg), and MONKEY 4 (M, 4.4 kg). Barium sulfate embedded caplets of formulation 2 in above primate absorption studies were orally administered to the primates. At given time points (0, 30, 60, 90 min. and 2.5h, 3h, 4h and 6h), X-ray pictures were taken to locate the caplets in the primates while blood samples were collected to measure heparin absorption (APTT/FXa). The dose levels were 30 mg/kg of heparin and 80 mg/kg of SNAC. Heparin absorption was assayed.

### Absorption study in primates on the bi-layered GRDF (Formulations 1 & 2)

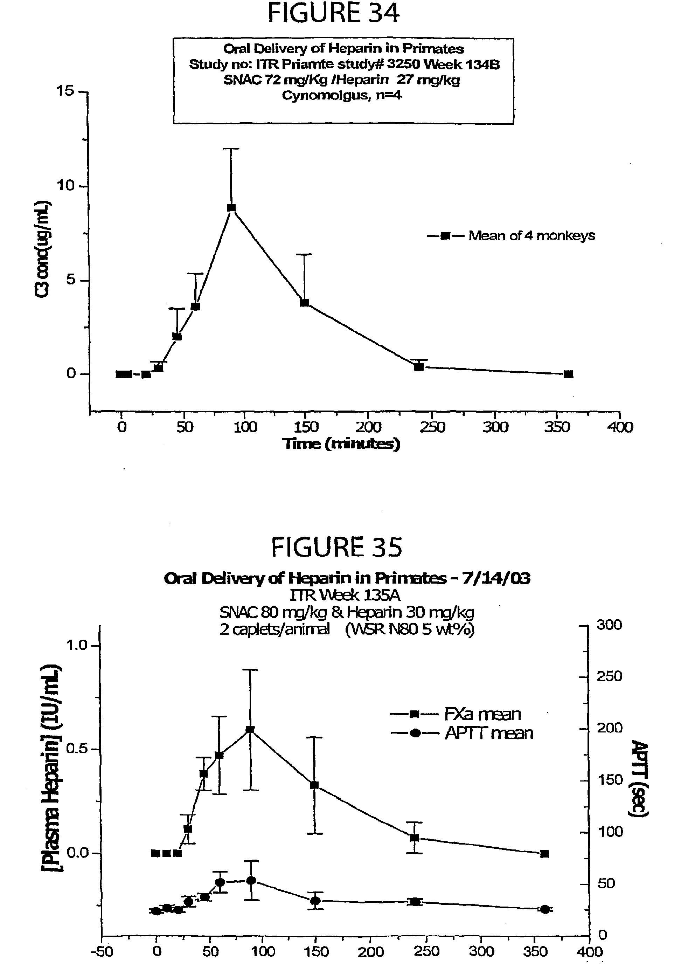

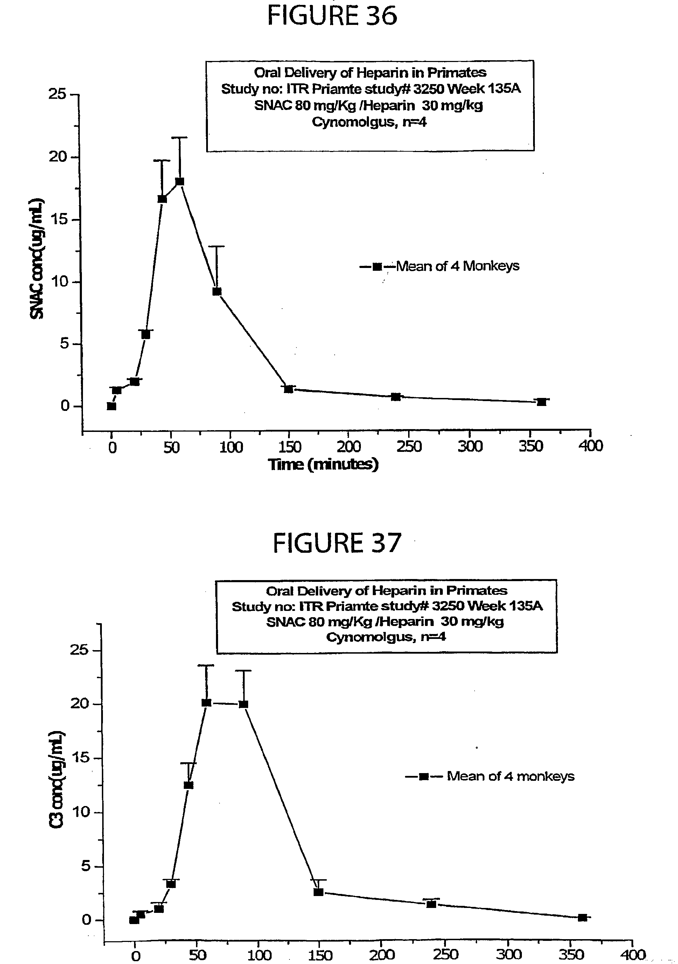

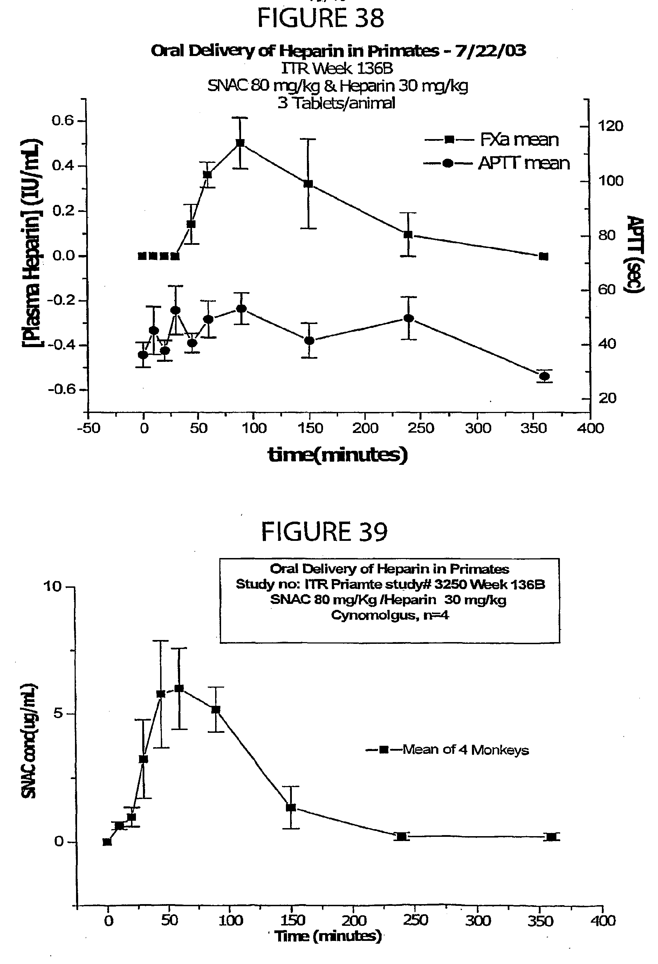

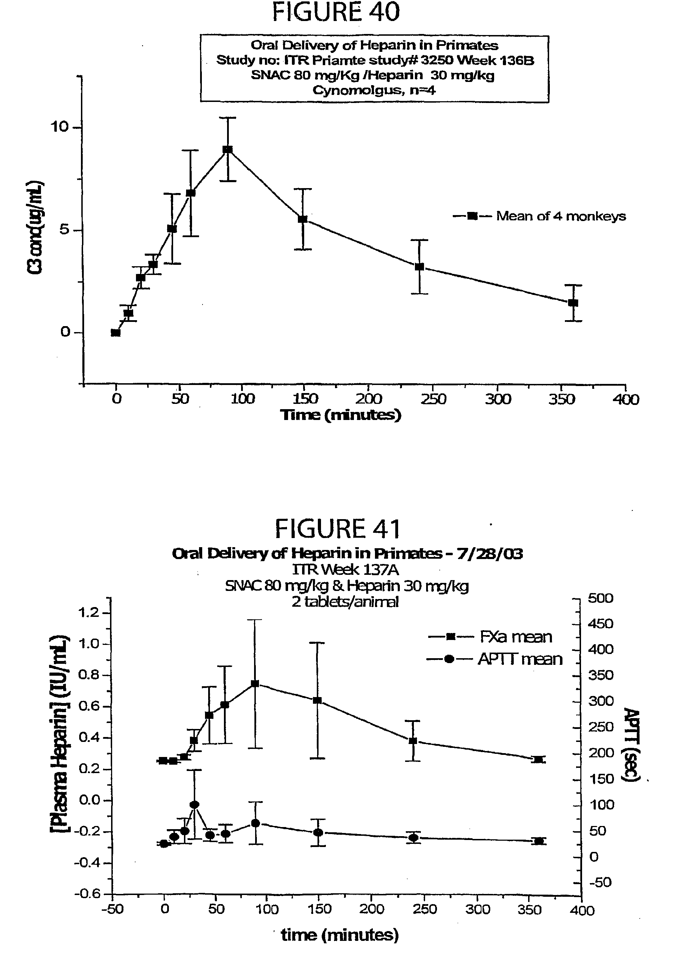

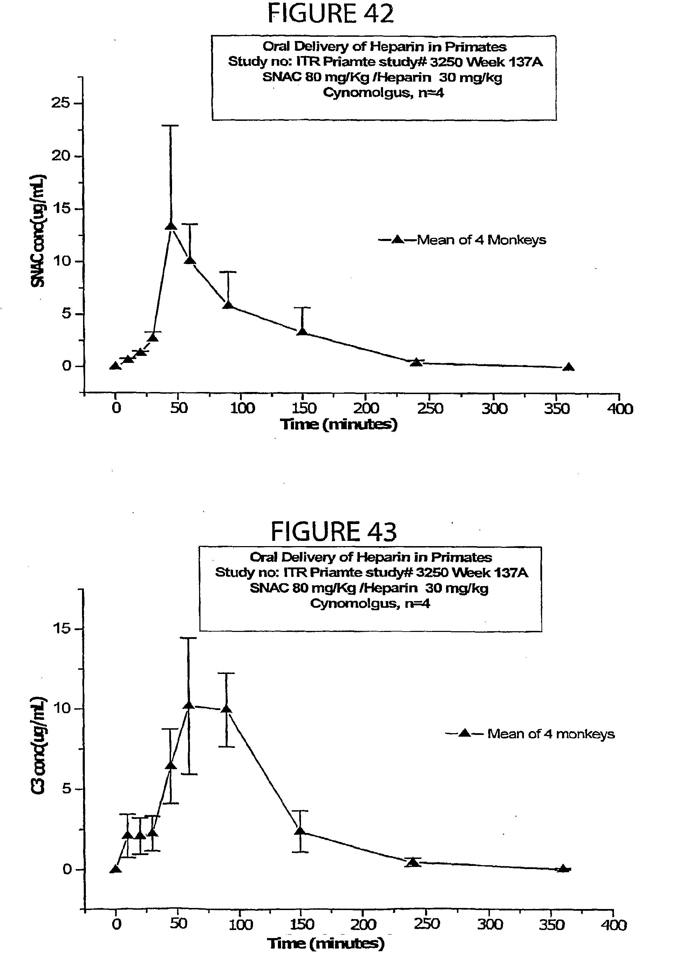

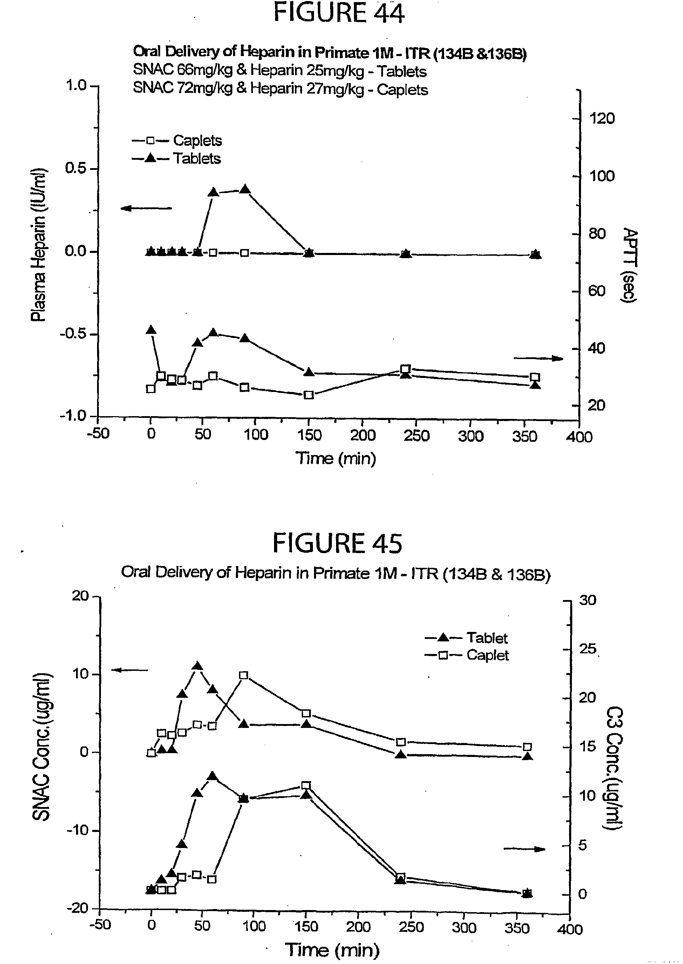

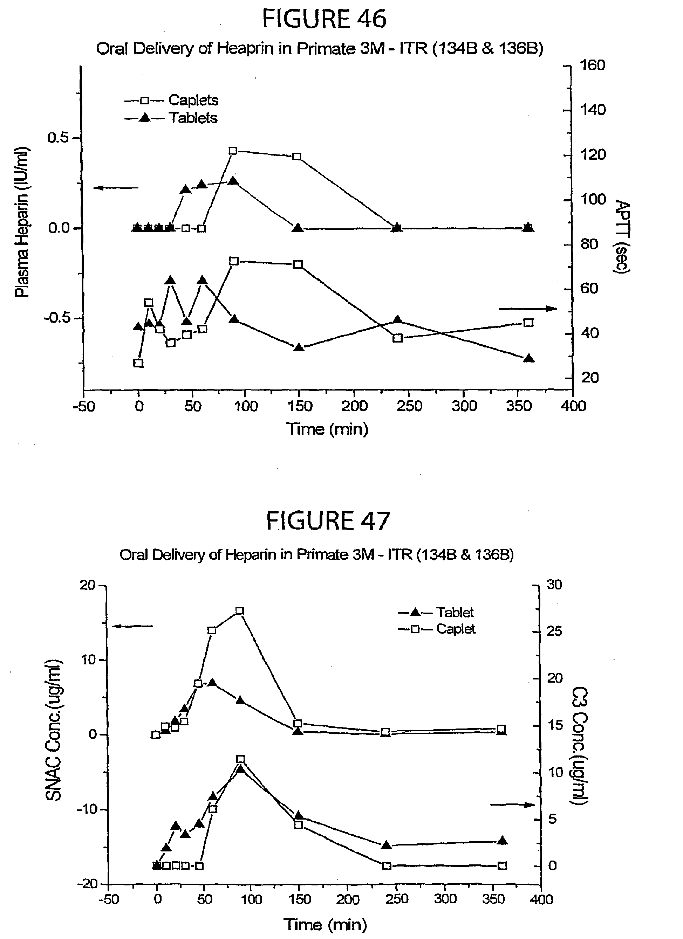

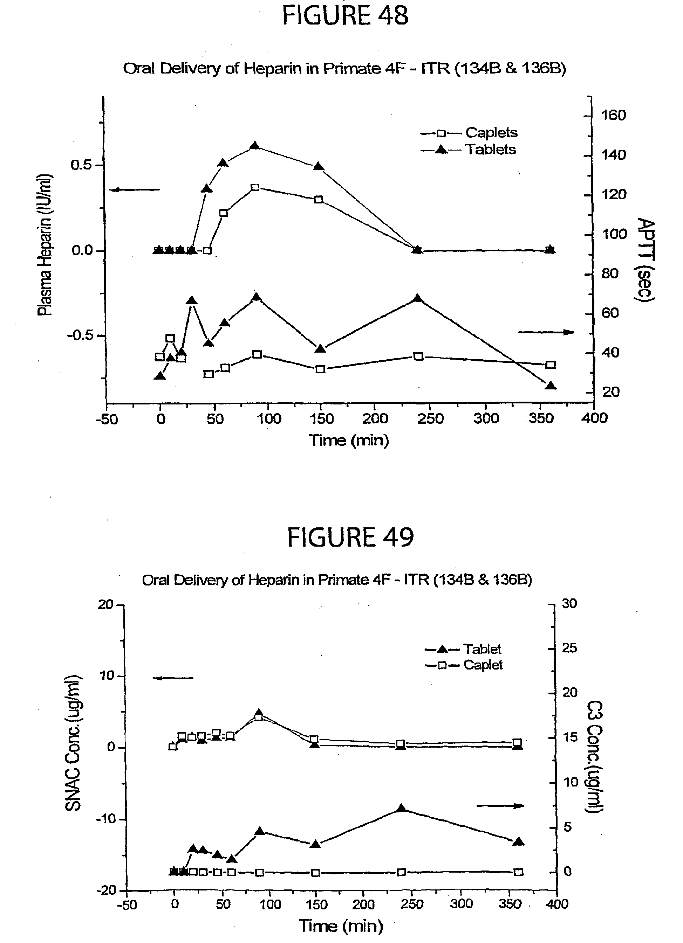

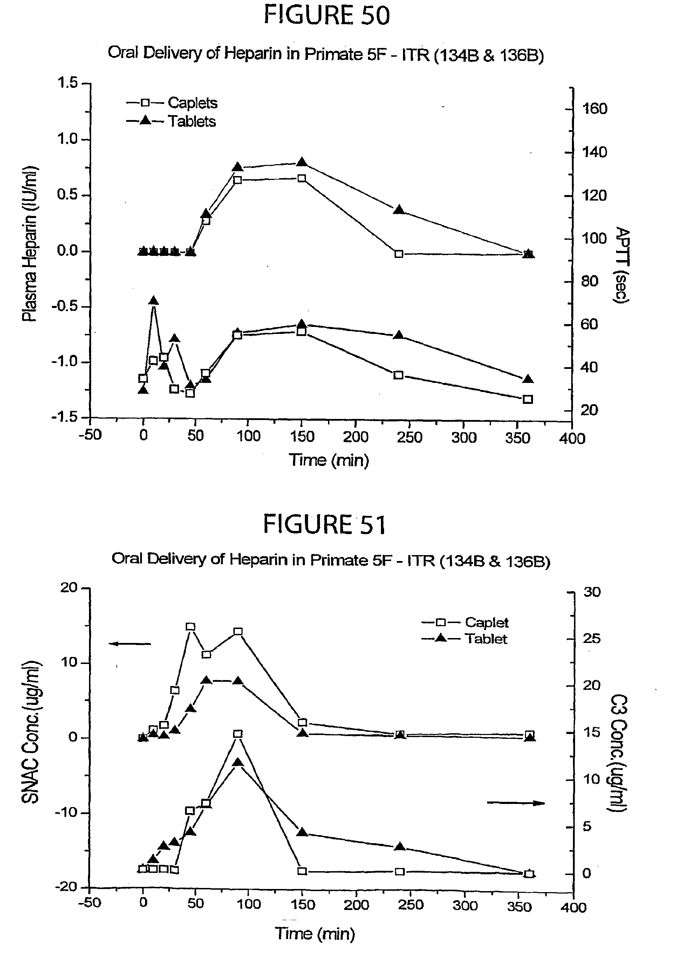

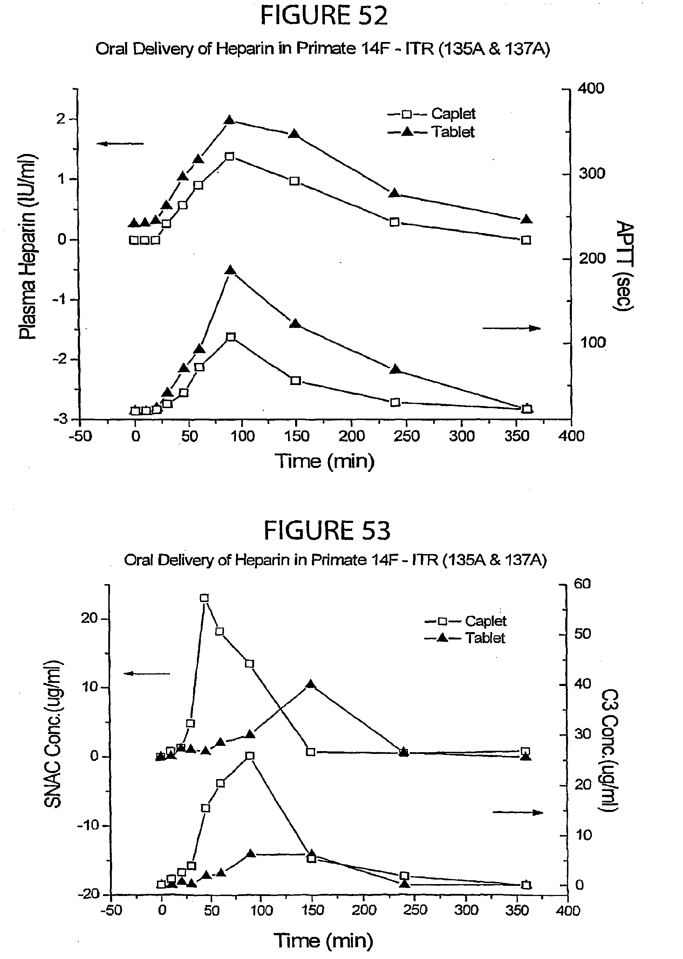

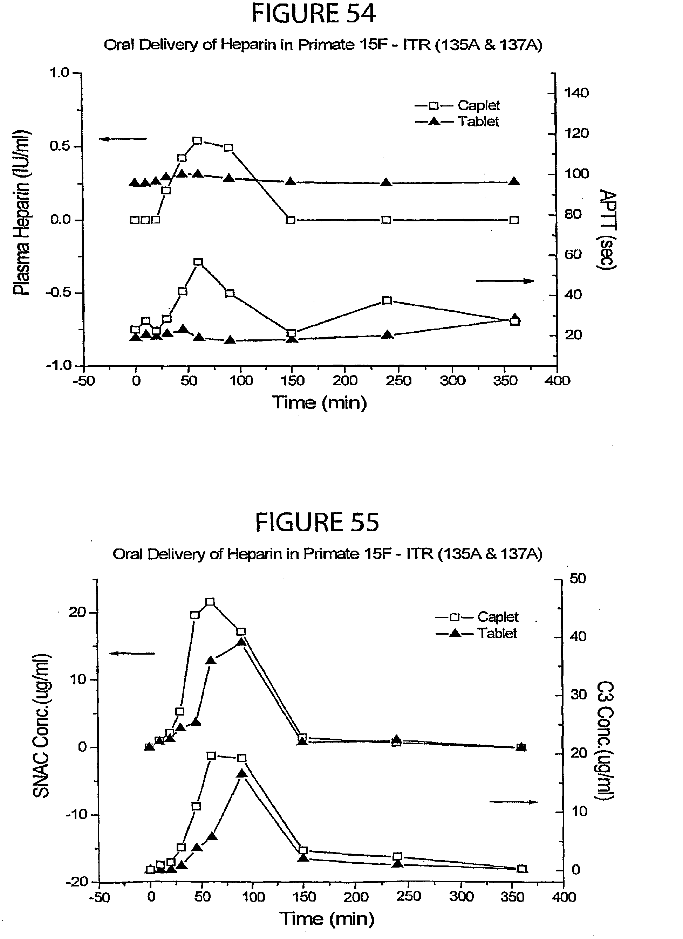

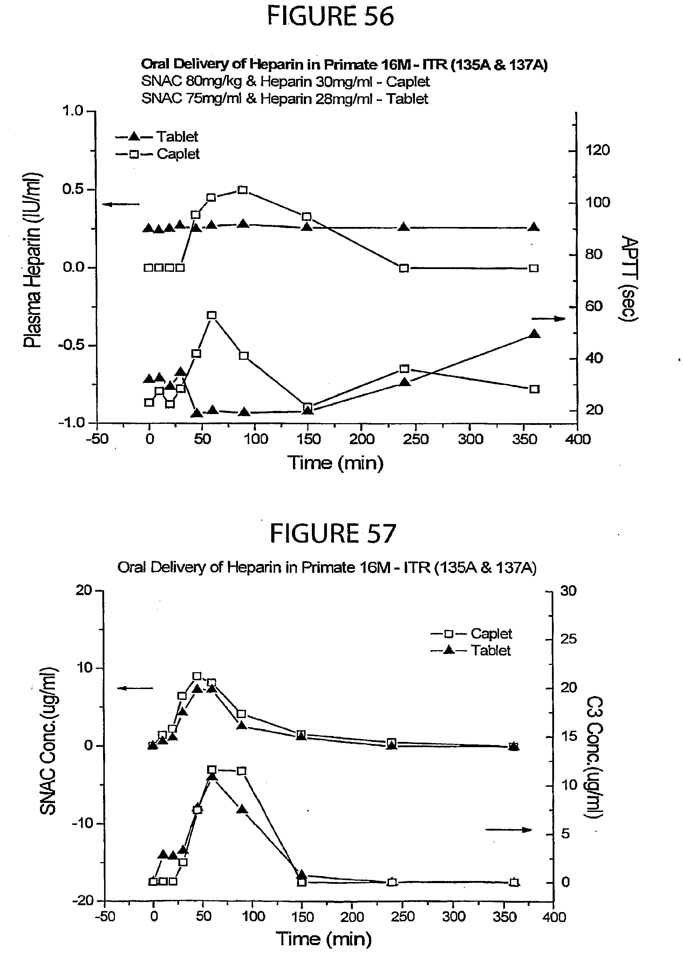

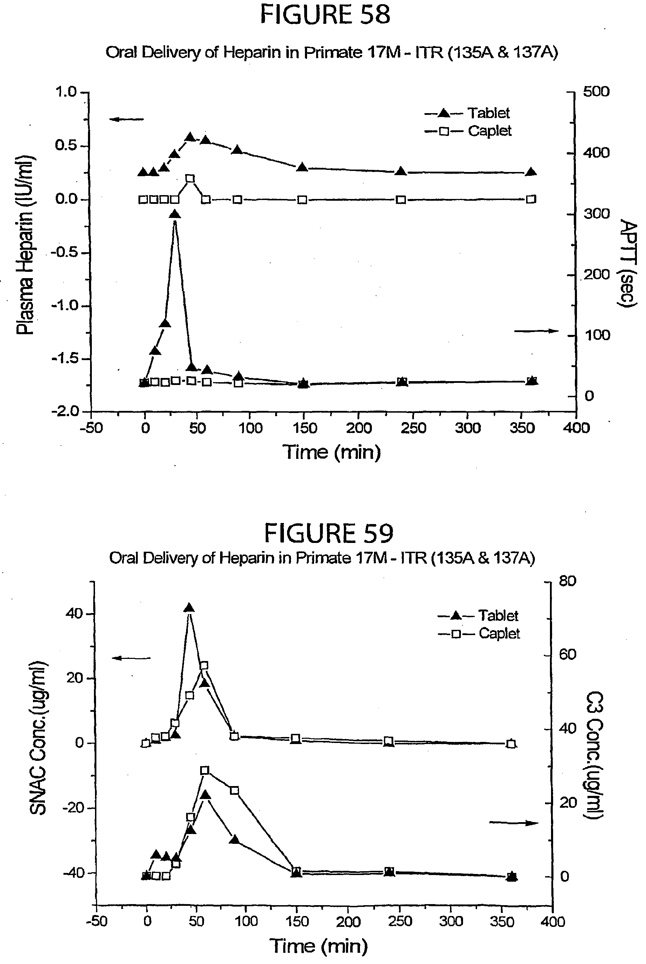

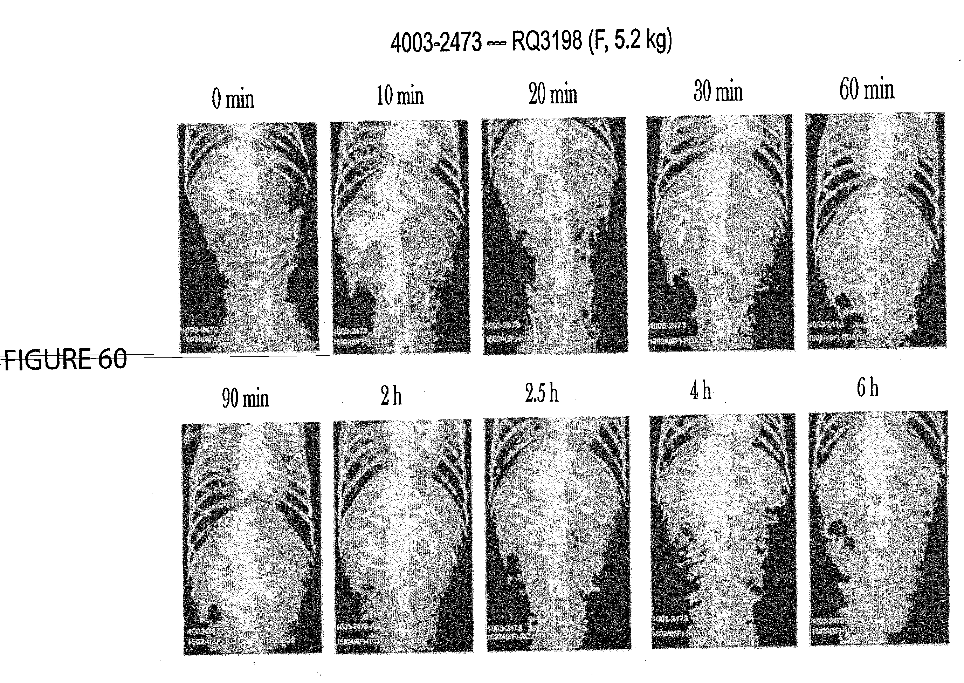

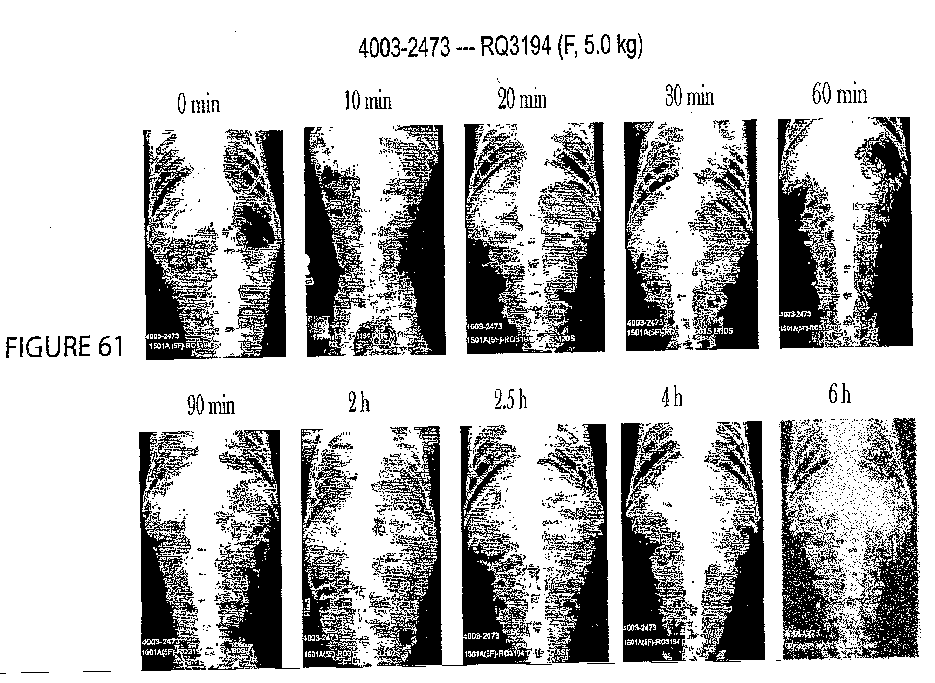

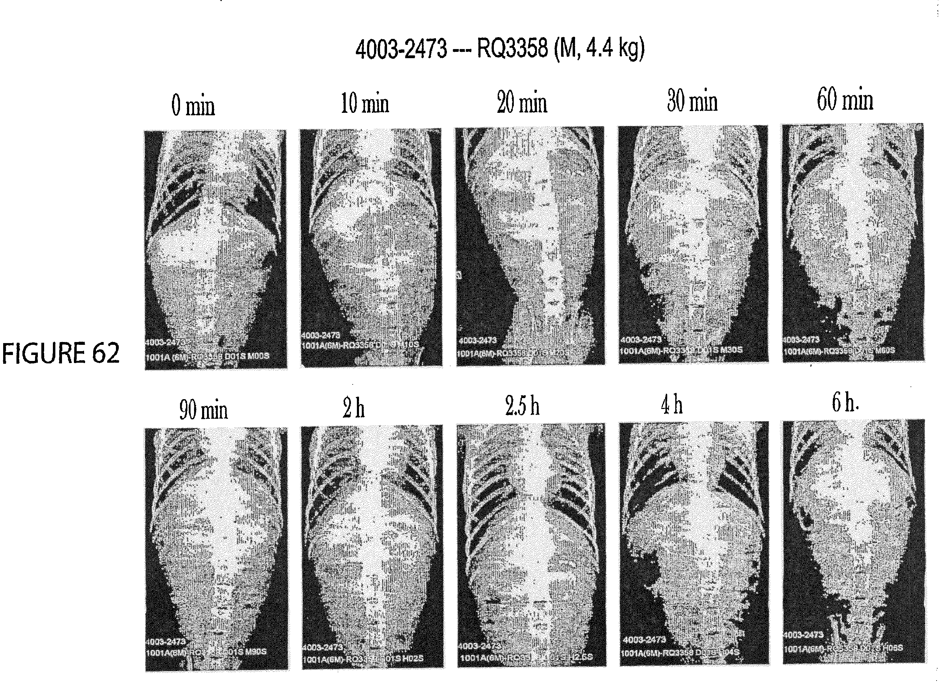

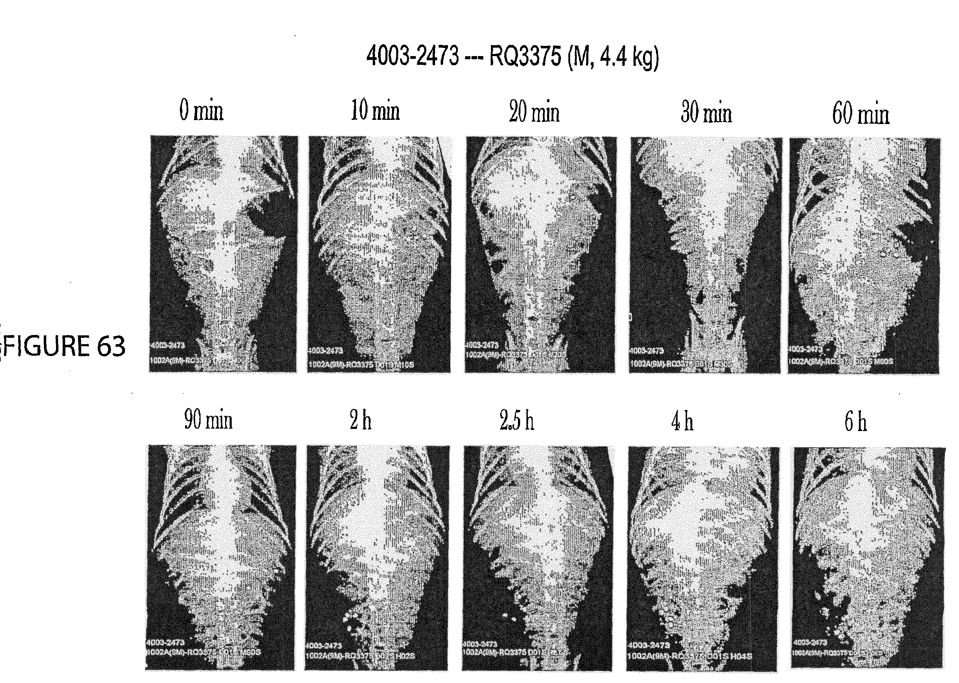

Two groups of crossover studies were performed in 4 primates on the two dosage forms, caplet and tablet, of two related formulations: Study A (formulation 1, caplets); Study B (tablets, formulation 1); Study C (formulation 2, caplets); and Study D (formulation 2, tablets). Experiments Study A /Study B and Study C /Study D were crossover, respectively. Both heparin and SNAC absorption were analyzed and the results were shown in Figures 29 - 40. The comparison profiles for the crossover experiment on individual monkeys were listed in Figures 41 - 56. C3 denotes the three carbon metabolite of SNAC.

Heparin / SNAC absorption profiles for all the four dosage forms were found different from that of our non-sustained release dosage forms (liquid and solid without polymer excipients). A delayed and a prolonged action time (up to 45min and 4h, respectively) were observed. Typically the Cmax (∼ 0.5 - 1 IU/ml for AntiFXa) were lower whereas the action times (∼ 4 - 6 h) were longer. The delayed action time, from 20 to 45min, depended on the content of the release controlling polymer polymer WSR N80 in the heparin/SNAC layer. When the amount of WSR N80 increased from 5 wt% to 13 wt%, the delayed action time increased from ∼20 min to ∼45 min. After 4 - 6 h, the heparin absorption quickly dropped to zero. This may be resulted from the clearance of the dosage forms from the stomach due to loss of their mechanical strength or just because of the complete release of heparin / SNAC during this time period.

To clarify this, a swelling test was thus performed with the caplet used for the primate study A. The data was shown in Table 7. It was observed that the caplets started to lose their mechanical strength after 3h. This might explain the sudden drop of heparin absorption to zero at 4h in the primate study A (Fig. 6).

**Table 7 Swelling of bi-layered caplets for primate experiment Study A (Formulation 1)**

| Time (min) | Length (mm) | Width (mm) | Heights (mm) | Strength |
|---|---|---|---|---|
| 0 | 15.5 | 5.79 | 6.32 | Strong |
| 15 | 16.75 | 7.13 | 8.58 | Strong |
| 30 | 17.36 | 7.92 | 8.81 | Strong |
| 45 | 18.25 | 8.60 | 8.60 | Strong |
| 60 | 18.85 | 9.25 | 8.50 | Strong |
| 105 | 20.92 | 10.02 | 9.32 | Strong |
| 180 | 22.0 | 10.40 | 9.12 | Strong/medium |
| 360 | 24.75 | 12.08 | 8.44 | Medium/weak |

Preliminary data analysis on the crossover primate study was performed and the results were shown in the following tables. As shown in table 8, for the same monkey, the tablet and the caplet have comparable Tmax, however, the tablet exhibited significantly higher (∼1.5 - 2 times) AUC than the caplet for all the four primates. This significance in the AUC difference is confirmed by the results of correlation variation analysis (Table 10), suggesting tablet is better than the caplet for formulation 2. But for formulation 1, it's in-conclusive which one is better (Table 9).

**Table 8 Heparin absorption (FXa) --- Formulation 2 (5% WSR N80)**

| Formulation 2 (5% WSR N80) | | | | | |
|---|---|---|---|---|---|
| Monkey | Prototype | AUC | Cmax | Tmax | Width (min) |
| 16M | Caplet | 62.6 | 0.5 | 90 | 195 |
| | Tablet | 94.3 | 0.28 | 90 | 360 |
| 17M | Caplet | 3 | 0.2 | 45 | 30 |
| | Tablet | 118.5 | 0.58 | 45 | 220 |
| 14F | Caplet | 200.1 | 1.39 | 90 | 180 |
| | Tablet | 381.2 | 1.98 | 90 | 195 |
| 15F | Caplet | 43 | 0.54 | 60 | 105 |
| | Tablet | 95.6 | 0.31 | 45 | 360 |
| Group | Caplet | 77.1 | 0.60 | 90 | 195 |
| Mean | Tablet | 172.4 | 0.75 | 90 | 330 |

**Table 9 Heparin absorption (FXa) --- Formulation 1 (13% WSR N80)**

| Formulation 1 (13% WSR N80) | | | | | |
|---|---|---|---|---|---|
| Monkey (min) | Prototype | AUC | Cmax | Tmax | Width |
| 1M | Caplet | 0 | 0 | -- | -- |
| | Tablet | 25.2 | 0.38 | 90 | 90 |
| 3M | Caplet | 49.5 | 0.43 | 90 | 180 |
| | Tablet | 20.2 | 0.26 | 90 | 105 |
| 4F | Caplet | 44.1 | 0.37 | 90 | 180 |
| | Tablet | 81.1 | 0.61 | 90 | 195 |
| 5F | Caplet | 85.8 | 0.67 | 150 | 150 |
| | Tablet | 143.5 | 0.81 | 150 | 150 |
| Group | Caplet | 44.8 | 0.36 | 90 | 180 |
| Mean | Tablet | 67.5 | 0.50 | 90 | 180 |

**Table 10 Tablet vs Caplet: Crossover study data analysis**

| Formulation 2 (5% WSR N80) | | | | | |
|---|---|---|---|---|---|
| SNAC+C3 | | | Heparin | | |
| Monkey | Prototype | AUC (Heparin) | AUC ratio (Tablet to Caplet) | AUC (SNAC+C3) | AUC ratio (Tablet to Caplet) |
| 16M | Caplet | 62.6 | 1 | 6907.8 | 0.82 |
| | Tablet | 94.3 | | 5696.4 | |
| 17M | Caplet | 3 | (39.5) | 14306.9 | 0.76 |
| | Tablet | 118.5 | | 10948.0 | |
| 14F | Caplet | 200.1 | 1.9 | 17059.5 | 0.44 |
| | Tablet | 381.2 | | 7572.4 | |
| 15F | Caplet | 43 | 2.2 | 15798.5 | 0.63 |
| | Tablet | 95.6 | | 9875.7 | |
| X±SD (CV) | Caplet | 77.2±85.6 (110%) | | 13518± 4548 (33.6%) | |
| | Tablet | 172.4±139.6 (81%) | | 8523± 2352 (27.6%) | |
| | | | 1.87±0.35 (18.8%) | | 0.66± 0.17(25.4%) |

### Preparation of the barium sulfate embedded bi-layered caplets

4.1562 g of WSR 303 and 41.6 mg of magnesium stearate were well mixed to form part A. 4.1083 g of SNAC/heparin co-dried powder (KF, 10.97%), 0.4156 g of WSR N80 and 41.6 mg of magnesium were well mixed to give part B. 0.2998 g of part A was weighed and added to the caplet die (size #2) as the first layer. Two pre-made barium sulfate beads of ∼35 mg were then embedded into the part A powder close to the surface in such a way that the two beads were well separated for easy recognition. 0.3261 g of the part B was then added to the top of the part A in the die as the second layer. The mixture was compressed under 1.5 ton pressures on a Carver press to form the caplet which contained 71.5 mg of heparin and 190 mg of SNAC. Based on two caplets per primate, the dose levels were 30mg/kg of heparin and 80 mg/kg of SNAC for a 4.75 kg primate.

### Gastric retention study in primates on the bi-layered caplet

A primate experiment was performed on four monkeys with BaSO₄ beads-embedded caplets of the above-discussed formulation to study the gastric retention of the caplets with X-ray monitoring. The size of the caplets was about size #1 capsule and two caplets were orally dosed into each of the four monkeys, corresponding to a dose level of 30 mg/kg of heparin and 80 mg/kg of SNAC. At given time points up to 6 hours, X-ray pictures were taken to locate the caplets in the primates while blood samples were collected to measure heparin absorption (APTT/FXa).

Both caplets dosed at time zero retained in the stomach for at least 6 hours (the maximum experimental time) on three of the four monkeys (MONKEY 2, MONKEY 1 and MONKEY 3). On the other monkey (MONKEY 4), the two caplets only stayed in the stomach for about 60 - 90 minutes and then exited to the small intestine.

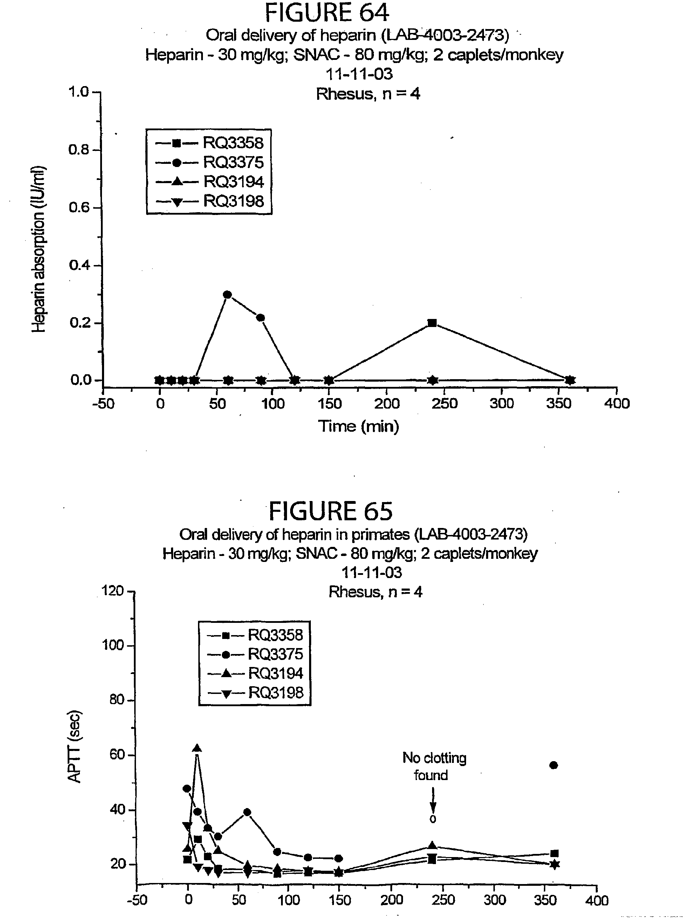

The corresponding heparin absorption results for the same primate study (n=4, Rhesus) were shown in Figures 64 & 65. Except for primate MONKEY 4, in which the caplets exited from the stomach at ∼ 60 to 90 min, no significant heparin absorption was observed for the other three primates. The blood anti-FXa level reached ∼ 0.3 IU/ml for primate MONKEY 4 at 60 min, corresponding to the Tmax of the APTT level of ∼ 40 seconds.

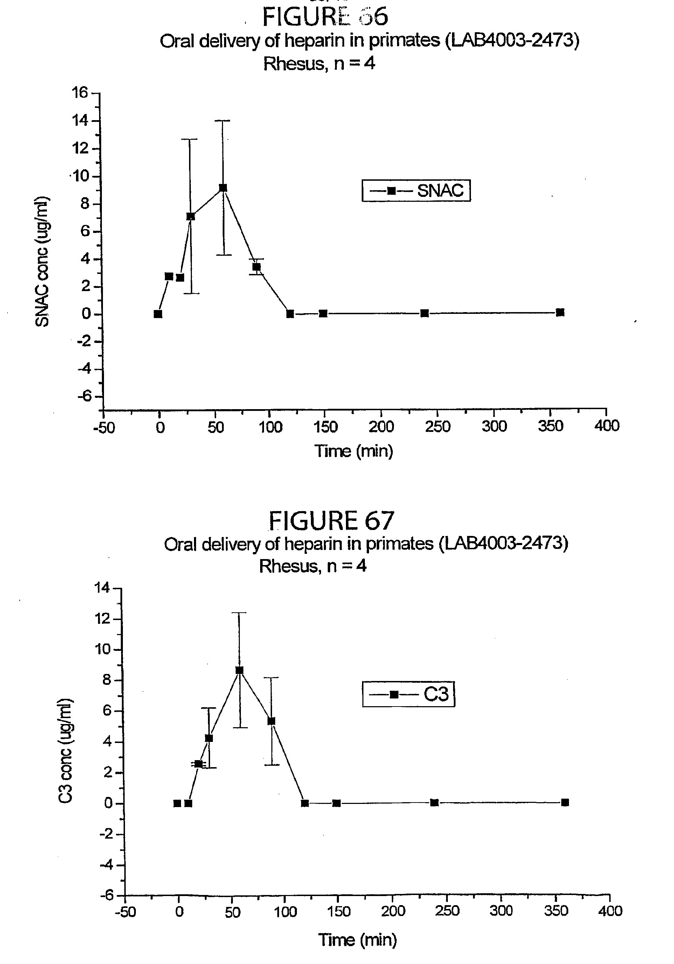

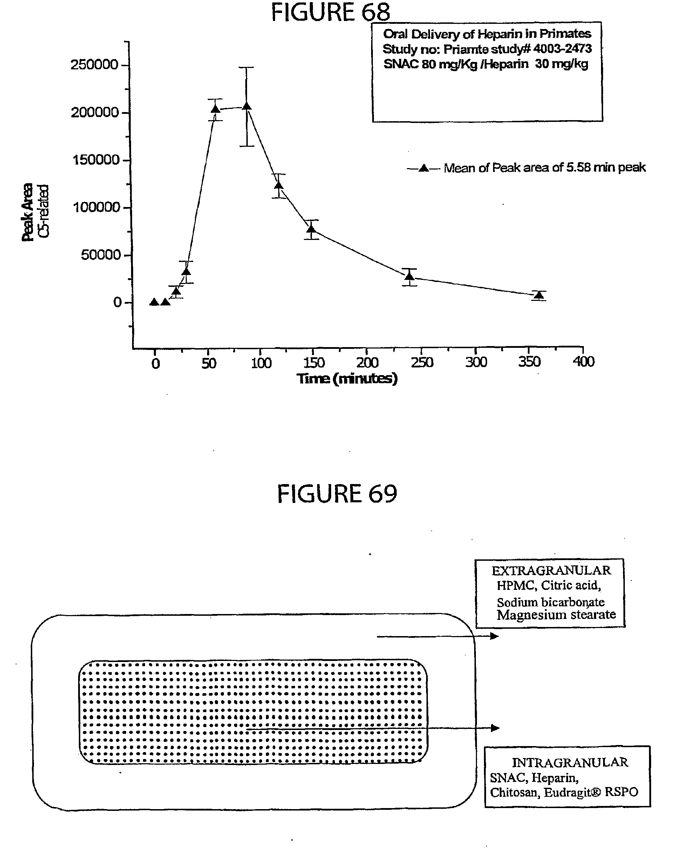

SNAC analysis results were also obtained (Figures 66 - 68). Significant SNAC and C3 absorption were observed for all the four primates. Unlike the heparin absorption situation, the SNAC / C3 absorption in primate MONKEY 4 was not the highest and the difference between this primate and other three primates was not that dramatic. A C5-related peak was observed during the SNAC/C3 analysis. Due to lack of the standard, it was not quantified (Figure 68)

### Example 8 Buoyancy, Primate Heparin delivery for Heparin/SNAC formulation

Two heparin/SNAC formulation was prepared based on the design set forth in Figure 69 having the formulation shown below:

| **Ingredients** | **Qty/tablet (mg)** | **Qty/tablet (mg)** |
|---|---|---|
| SNAC | 230 | 459.96 |
| Heparin | 85.25 | 170.49 |
| Chitosan | 4.95 | 9.90 |
| Eudragit® RSPO | 6.6 | 13.20 |
| Methocel(Internal) | 0.88 | 1.76 |
| Water | 14.24 | 28.47 |
| Methocel (External) 20% | 99.1 | 198.2 |
| Citric acid (5%) | 24.78 | 49.55 |
| Sodium Bicarbonate (5%) | 24.78 | 49.55 |
| Mag Stearate (1%) | 4.96 | 9.91 |
| Total | 495.5 | 990.99 |

SNAC, Heparin, Chitosan and Eudragit® RSPO were prepared via a wet granulation process to produce the intragranular core, and Methocel, Citric Acid, sodium bicarbonate and magnesium stearate were applied extragranularly. The 495.5 mg tablet was pressed to a hardness of 9kp, and the 990.99mg was pressed to a hardness of 8 kp.

Flotation tests were performed on each tablet, shown below:

| Formulation | Float duration in SGF (pH 1.2) at 37°C (Float lag time) | Float duration in SGF at Room Temperature (Float lag time) | Float duration in Water at 37°C (Float lag time | Float duration in Water at Room Temperature (Float lag time) |
|---|---|---|---|---|
| 495 mg tablet (hardness = 9 kp | greater than 8 hours (less than 15 second lag time) | greater than 8 hours (2-3 minutes lag time) | Did not float | Did not float |
| 990 mg tablet (hardness = 8 kp) | greater than 8 hours | greater than 8 hours | Did not float | Did not float |

Trials were later run to determine that a hardness less than 3 kp was required in order for the above caplets to float in water.

The buoyancy of the tablets in the acidic medium of simulated gastric fluid, but not water, indicates that floating behavior is dependent upon the reaction of the sodium bicarbonate and the acidic test medium.

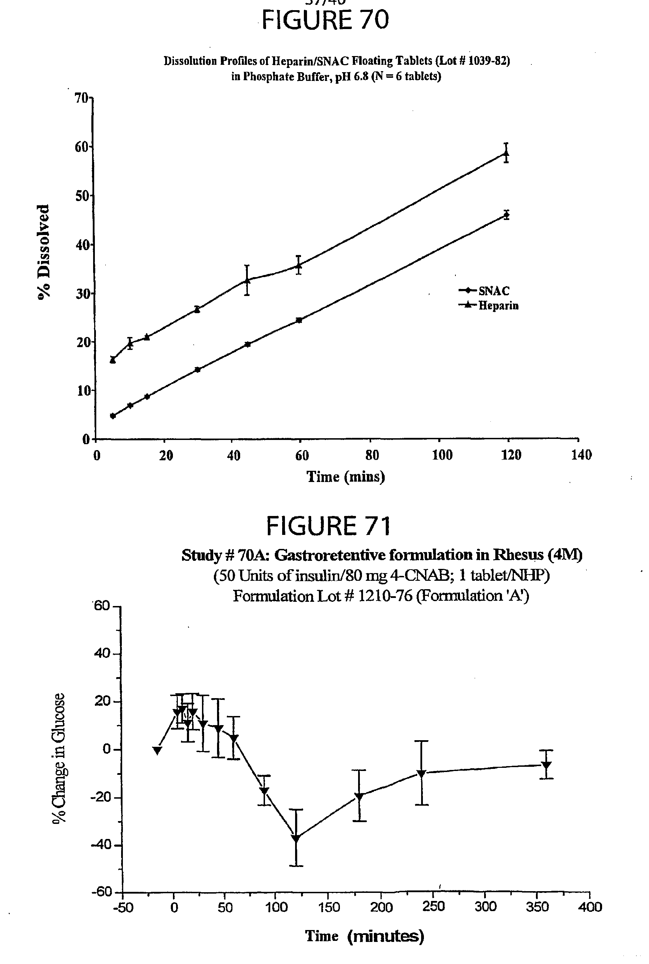

Dissolution profiles, based on % dissolved, were obtained for SNAC and heparin over 2 hours in Phosphate buffer (pH = 6.8). The results are set forth in Figure 70.

The above formulations were orally administered to Rhesus monkeys. Plasma heparin concentrations were not observed over a period of 350 minutes. It is believed that the presence of the sodium bicarbonate inhibited the absorption of heparin.

### Example 9 4-CNAB/Insulin Gastric Retention Delivery System

The following two formulations were prepared with a wet granulation/extragranual design analogous to Figure 69 and pressed into tablets:

| | **Formulation A** | **Formulation B** |
|---|---|---|
| **Ingredients** | **Qty/tablet** (**mg**) | **Qty/tablet (mg)** |
| 4-CNAB | 80.64 | 80.64 |
| Insulin | 1.82 (50 units) | 1.82 (50 units) |
| Chitosan (1.5%w/w) | 1.27 | 1.41 |
| Methocel (Internal) | 0.84 | 0.94 |
| Sodium Alginate | 0 | 9.42 |
| Water | 2.52 | 2.64 |
| Methocel (External) - 20% | trace | 25.52 |
| Mag Stearate (1%) | trace | 1.23 |
| Total | 87.1 | 122.62 |

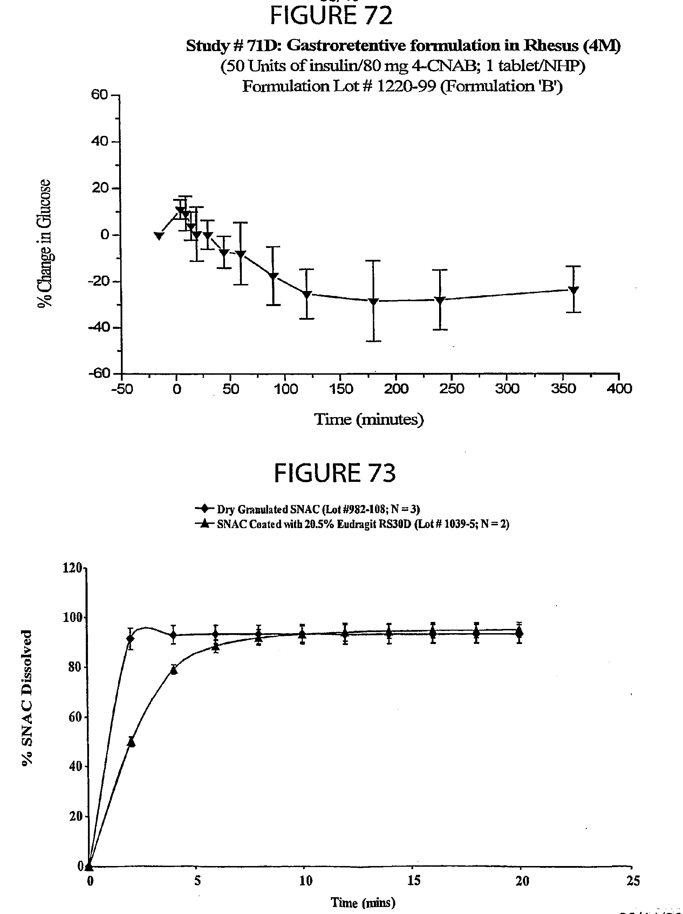

Formulations A and B were administered by oral gavage to the same group of four male Rhesus monkeys and the percent reduction in glucose was obtained over a period of about 6 hours. The averaged results for Formulation A is shown in Figure 71 and the results for Formulation B is shown in Figure 72.

### Example 10 Retardation of SNAC Disolution via addition of Eudragits

The dissolution of SNAC was investigated in formulations coated or granulated with Eudragit RS30D (poly(ethylacrylate, methylmethacrylate, trimethylammonioethyl methacrylate) chloride)) or dry SNAC granules blended with 33.4% Eudragit RSPO. The Eudragit line of products is available from Rohm Pharma GmbH Westerstadt, Germany.

The following SNAC formulations were prepared:
Formulation 1: Dry granulated SNAC granules
Formulation 2: Dry SNAC granules coated with 20.5% Eduragit RS30D
Formulation 3: Dry Granules of SNAC
Formulation 4: SNAC granulated with 12.6% Eudragit RS30D
Formulation 5: SNAC granulated with 33.4% Eudragit RS30D
Formulation 6: Dry granules of SNAC blended with 33.4% Eudragit RSPO

The dissolution rates of the above SNAC formulations, based on % dissolved, were obtained in Phosphate buffer having a PH of 6.8 are set forth in the table below:

| Formulation | Dissolution rate (mg/min) |
|---|---|
| 1 | 11.18 ± 11.53 (n=3) |
| 2 | 46.21 ± 1.95 (n=2) |
| 3 | 24.50 ± 1.07 (n=3) |
| 4 | 18.31 ± 0.47 (n=3) |
| 5 | 2.93 ± 0.09 (n=3) |
| 6 | 12.68 ± 1.86 (n-3) |

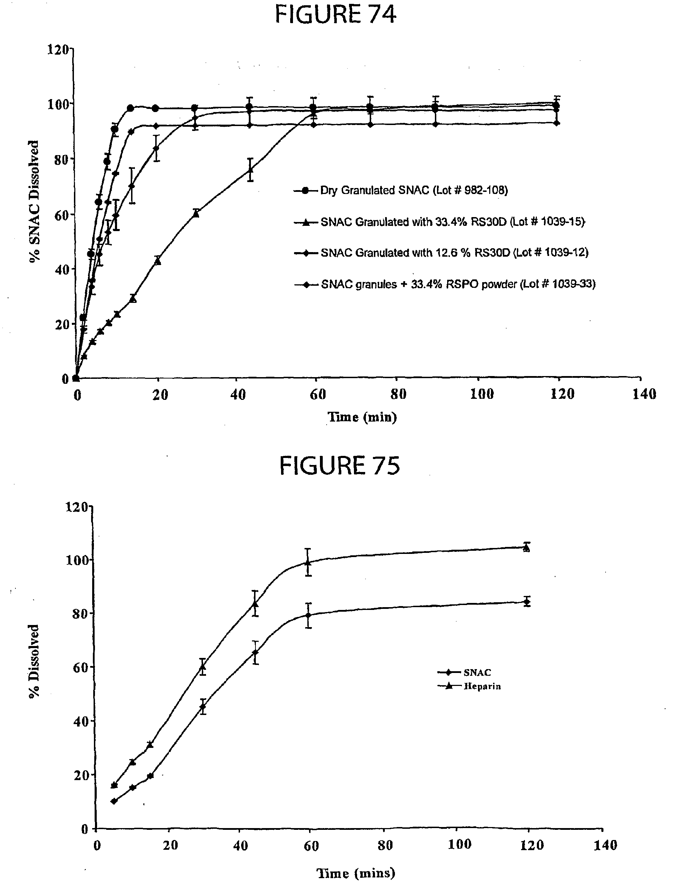

A plot comparing the dissolution time of Formulation 1 versus Formulation 2 is shown in Figure 73. A plot comparing Formulations 1, 4, 5 and 6 is shown in Figure 74.

### Example 11: Controlled Release Formulation of Heparin/SNAC

A controlled release formulation was prepared by granulating a portion of the delivery agent SNAC with 33.4% Eudragit RS30D. This is referred to as "Slow Dissolving SNAC" in the chart below. SNAC was also added in the form of dry granulated Granules, and is referred to as "Fast Dissolving SNAC". An immediate release formulation was also prepared, in which all of the SNAC was "Fast Dissolving SNAC", i.e., added in the form of dry granulated SNAC granules. The ingredients of each formulation are set forth below:

| Ingredients | Immediate Release Formulation (mg/tablet) | Controlled Release Formulation (mg/tablet) |
|---|---|---|
| Heparin | 170.5 | 170.5 |
| "Fast dissolving" SNAC | 460.0 | 153.33 |
| "Slow dissolving"SNAC | 0 | 306.67 |
| Emcompress, Internal (10%) | 80.0 | 100.0 |
| SLS (1%) | 8.0 | 10.0 |
| Water | 25.28 | 20.97 |
| Eudragit® RS30D | 0 | 153.33 |
| Emcompress (External) | 46.7 | 73.17 |
| Cab-O-Sil® (0.2%) | 1.60 | 2.0 |
| Magnesium Stearate (1%) | 8.0 | 10.0 |
| TOTAL | 800.08 | 1000.0 |

The Heparin, SNAC (with and without Eudragit RS30D) Emcompress, Sodium Lauryl Sulfate, and water were formulated via wet granulation to form an inner core. Emcompress, Cab-O-Sil, and Magnesium Stearate to form the extragranular outer portion. The intragranular and extragranular cores were pressed to form a tablet.

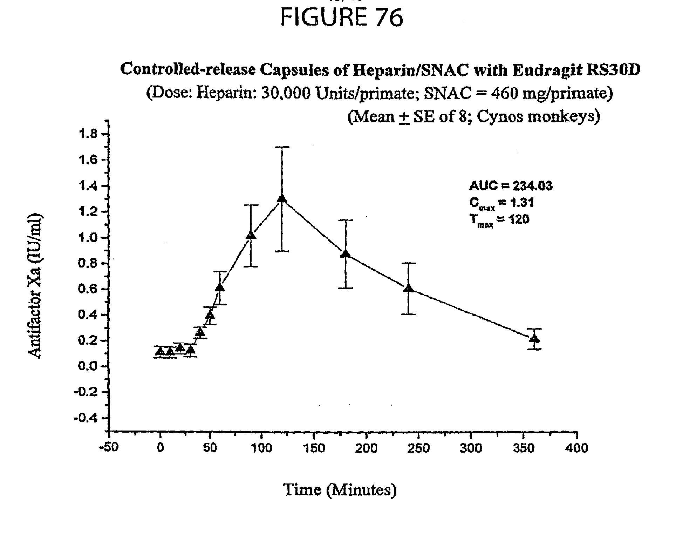

The dissolution profile of the SNAC and heparin in the controlled release formulation is shown in Figure 75. The Antifactor Xa activity of the controlled release formulation when administered to 8 Cynos monkeys, measured over a period of 6 hours, is shown in Figure 76.

## Claims

1. A bi-layered pharmaceutical composition comprising:
(i) one layer comprising a biologically active agent, a delivery agent compound, a release controlling polymer; and
(ii) a second layer comprising a swellable polymer;
wherein the swellable polymer has a molecular weight of at least 50,000 g/mol (50,000 daltons), wherein the delivery agent compound is N-(8-[2-hydroxybenzoyl]-amino) caprylic acid, or a pharmaceutically acceptable salt thereof, and wherein the active agent is heparin.

2. The pharmaceutical composition of claim 1, wherein the swellable polymer is selected from a crosslinked poly(acrylic acid), a poly(alkylene oxide), a poly(vinyl alcohol), a poly(vinyl pyrrolidone), a polyurethane hydrogel, a maleic anhydride polymer, a cellulose polymer, a polysaccharide, a starch, and a starch based polymer.

3. The pharmaceutical composition of claim 1, wherein the swellable polymer is a poly(alkylene oxide).

4. The pharmaceutical composition of claim 3, wherein the poly(alkylene oxide) is a polymer containing at least one of ethylene oxide or propylene oxide as a monomer unit.

5. The pharmaceutical composition of claim 1, wherein the swellable polymer is a poly(ethylene oxide) having a molecular weight in excess of 500,000 g/mol (500,000 daltons).

6. The pharmaceutical composition of any one of the preceding claims, wherein the swellable polymer is a polyethylene oxide having an average molecular weight of 7,000,000 g/mol (7,000,000 daltons).

7. The pharmaceutical composition of claim 1, wherein the release controlling polymer is selected from a poly(ethylene oxide), a poly(acrylic acid), a poly(acrylate), a polyvinyl alcohol, an alginate, a chitosan, a polyvinylpyrrolidone, a cellulose polymer and a polysaccharide.

8. The pharmaceutical composition of claim 1, wherein the release controlling polymer is a poly(ethylene oxide) having a molecular weight of 300,000 g/mol (300,000 daltons).

9. The pharmaceutical composition of claim 1, wherein the release controlling polymer is a poly(ethylene oxide) having a molecular weight of 200,000 g/mol (200,000 daltons).

10. The pharmaceutical composition of claim 1, wherein the release controlling polymer is a poly(acrylic acid) or a poly(acrylate).

11. The pharmaceutical composition of claim 1, wherein the active agent's absorption begins 15 to 30 minutes from administration and lasts up to 6.0 hours after oral administration to a mammal.

12. The pharmaceutical composition of claim 1, wherein the composition increases in size by at least 10 - 15% while in the stomach within about 30 minutes of oral administration to a mammal.

13. The pharmaceutical composition of claim 1, wherein the delivery agent is the monosodium salt of N-(8-[2-hydroxybenzoyl]amino) caprylic acid.

14. The pharmaceutical composition of any one of the preceding claims, wherein the pharmaceutical composition is a caplet or a tablet.

## Patentansprüche

1. Doppellagige pharmazeutische Zusammensetzung, umfassend:
(i) eine Lage, umfassend ein biologisch aktives Mittel, eine Verabreichungsmittelverbindung, ein die Freisetzung kontrollierendes Polymer; und
(ii) eine zweite Lage, umfassend ein quellbares Polymer;
wobei das quellbare Polymer ein Molekulargewicht von mindestens 50.000 g/mol (50.000 Dalton) besitzt, wobei die Verabreichungsmittelverbindung N-(8-[2-Hydroxybenzoyl]-amino)caprylsäure oder ein pharmazeutisch verträgliches Salz davon ist und wobei das aktive Mittel Heparin ist.

2. Pharmazeutische Zusammensetzung nach Anspruch 1, wobei das quellbare Polymer ausgewählt ist aus einer quervernetzten Poly(acrylsäure), einem Poly(alkylenoxid), einem Poly(vinylalkohol), einem Poly(vinylpyrrolidon), einem Polyurethanhydrogel, einem Maleinsäureanhydridpolymer, einem Cellulosepolymer, einem Polysaccharid, einer Stärke und einem stärkebasierten Polymer.

3. Pharmazeutische Zusammensetzung nach Anspruch 1, wobei das quellbare Polymer ein Poly(alkylenoxid) ist.

4. Pharmazeutische Zusammensetzung nach Anspruch 3, wobei das Poly(alkylenoxid) ein Polymer ist, das mindestens eines von Ethylenoxid oder Propylenoxid als Monomereinheit enthält.

5. Pharmazeutische Zusammensetzung nach Anspruch 1, wobei das quellbare Polymer ein Poly(ethylenoxid) mit einem Molekulargewicht von mehr als 500.000 g/mol (500.000 Dalton) ist.

6. Pharmazeutische Zusammensetzung nach einem der vorangegangenen Ansprüche, wobei das quellbare Polymer ein Polyethylenoxid mit einem durchschnittlichen Molekulargewicht von 7.000.000 g/mol (7.000.000 Dalton) ist.

7. Pharmazeutische Zusammensetzung nach Anspruch 1, wobei das die Freisetzung kontrollierende Polymer ausgewählt ist aus einem Poly(ethylenoxid), einer Poly(acrylsäure), einem Poly(acrylat), einem Polyvinylalkohol, einem Alginat, einem Chitosan, einem Polyvinylpyrrolidon, einem Cellulosepolymer und einem Polysaccharid.

8. Pharmazeutische Zusammensetzung nach Anspruch 1, wobei das die Freisetzung kontrollierende Polymer ein Poly(ethylenoxid) mit einem Molekulargewicht von 300.000 g/mol (300.000 Dalton) ist.

9. Pharmazeutische Zusammensetzung nach Anspruch 1, wobei das die Freisetzung kontrollierende Polymer ein Poly(ethylenoxid) mit einem Molekulargewicht von 200.000 g/mol (200.000 Dalton) ist.

10. Pharmazeutische Zusammensetzung nach Anspruch 1, wobei das die Freisetzung kontrollierende Polymer eine Poly(acrylsäure) oder ein Poly(acrylat) ist.

11. Pharmazeutische Zusammensetzung nach Anspruch 1, wobei die Absorption des aktiven Mittels 15 bis 30 Minuten nach Verabreichung beginnt und bis zu 6,0 Stunden anhält nach oraler Verabreichung an einem Säuger.

12. Pharmazeutische Zusammensetzung nach Anspruch 1, wobei die Zusammensetzung ihre Größe um mindestens 10 - 15 % vergrößert, während sie im Magen ist, innerhalb von etwa 30 Minuten nach oraler Verabreichung an einen Säuger.

13. Pharmazeutische Zusammensetzung nach Anspruch 1, wobei das Verabreichungsmittel das Mononatriumsalz von N-(8-[2-Hydroxybenzoyl]amino)caprylsäure ist.

14. Pharmazeutische Zusammensetzung nach einem der vorangegangenen Ansprüche, wobei die pharmazeutische Zusammensetzung eine Filmtablette oder eine Tablette ist.

## Revendications

1. Composition pharmaceutique à deux couches comprenant :
(i) une couche comprenant un agent biologiquement actif, un composé agent d'administration, un polymère de contrôle de libération ; et
(ii) une deuxième couche comprenant un polymère gonflable ; dans laquelle le polymère gonflable a une masse moléculaire d'au moins 50 000 g/mol (50 000 daltons),
dans laquelle le composé agent d'administration est l'acide N-(8-[2-hydroxybenzoyl]amino)caprylique, ou un sel pharmaceutiquement acceptable de celui-ci, et dans laquelle l'agent actif est l'héparine.

2. Composition pharmaceutique de la revendication 1, dans laquelle le polymère gonflable est choisi parmi un poly(acide acrylique) réticulé, un poly(oxyde d'alkylène), un poly(alcool vinylique), une poly(vinylpyrrolidone), un hydrogel de polyuréthane, un polymère d'anhydride maléique, un polymère de cellulose, un polysaccharide, un amidon, et un polymère à base d'amidon.

3. Composition pharmaceutique de la revendication 1, dans laquelle le polymère gonflable est un poly(oxyde d'alkylène).

4. Composition pharmaceutique de la revendication 3, dans laquelle le poly(oxyde d'alkylène) est un polymère contenant au moins un oxyde d'éthylène ou oxyde de propylène en tant que motif monomère.

5. Composition pharmaceutique de la revendication 1, dans laquelle le polymère gonflable est un poly(oxyde d'éthylène) ayant une masse moléculaire supérieure à 500 000 g/mol (500 000 daltons).

6. Composition pharmaceutique de l'une quelconque des revendications précédentes, dans laquelle le polymère gonflable est un oxyde de polyéthylène ayant une masse moléculaire moyenne de 7 000 000 g/mol (7 000 000 daltons).

7. Composition pharmaceutique de la revendication 1, dans laquelle le polymère de contrôle de libération est choisi parmi un poly(oxyde d'éthylène), un poly(acide acrylique), un poly(acrylate), un alcool polyvinylique, un alginate, un chitosane, une polyvinylpyrrolidone, un polymère de cellulose et un polysaccharide.

8. Composition pharmaceutique de la revendication 1, dans laquelle le polymère de contrôle de libération est un poly(oxyde d'éthylène) ayant une masse moléculaire de 300 000 g/mol (300 000 daltons).

9. Composition pharmaceutique de la revendication 1, dans laquelle le polymère de contrôle de libération est un poly(oxyde d'éthylène) ayant une masse moléculaire de 200 000 g/mol (200 000 daltons).

10. Composition pharmaceutique de la revendication 1, dans laquelle le polymère de contrôle de libération est un poly(acide acrylique) ou un poly(acrylate).

11. Composition pharmaceutique de la revendication 1, dans laquelle l'absorption de l'agent actif commence 15 à 30 minutes à compter de l'administration et dure jusqu'à 6,0 heures après l'administration orale à un mammifère.

12. Composition pharmaceutique de la revendication 1, laquelle composition a sa taille qui augmente d'au moins 10 à 15 % tandis qu'elle est dans l'estomac depuis moins de 30 minutes environ à compter de l'administration orale à un mammifère.

13. Composition pharmaceutique de la revendication 1, dans laquelle l'agent d'administration est le sel monosodique de l'acide N-(8-[2-hydroxybenzoyl]amino)caprylique.

14. Composition pharmaceutique de l'une quelconque des revendications précédentes, laquelle composition pharmaceutique est un comprimé-capsule ou un comprimé.
